# EUROPÄISCHE PATENTSCHRIFT

(11) **EP 2 868 593 B1**
(45) Veröffentlichungstag und Bekanntmachung des Hinweises auf die Patenterteilung: **21.12.2016**
(21) Anmeldenummer: 14189573.0
(22) Anmeldetag: 20.10.2014
(51) Int. Cl.: B65D 25/10, A61M 5/00, B01L 9/06, B65D 77/04, B65D 85/42

(54) **Haltestruktur zum gleichzeitigen Halten einer Mehrzahl von Behältern für Substanzen für medizinische, pharmazeutische oder kosmetische Anwendungen sowie Transport- und Verpackungsbehälter mit Selbiger sowie Verfahren**
Support structure for simultaneously holding a plurality of containers for substances for medical, pharmaceutical or cosmetic applications and transport and packaging containers with same and method
Structure de support pour le support simultané d'une pluralité de récipients pour des substances pour des applications médicales, pharmaceutiques ou cosmétiques, récipient de transport ou d'emballage doté de celle-ci et procédé

(30) Priorität: 05.11.2013 DE 102013112167
(43) Veröffentlichungstag der Anmeldung: 06.05.2015
(73) Patentinhaber: Schott AG, 55122 Mainz (DE)
(72) Erfinder: Deutschle, Gregor Fritz, 65185 Wiesbaden (DE); Pawlowski, Edgar, 55271 Stadecken-Elsheim (DE); Jörn, Wassenberg, 55130 Mainz (DE); Reisse, Andreas, 93057 Regensburg (DE)
(74) Vertreter: 2K Patentanwälte Blasberg Kewitz & Reichel

(56) Entgegenhaltungen:
- WO-A1-2009/015862
- WO-A1-2011/135085
- WO-A1-2012/143533

## Beschreibung

### Gebiet der Erfindung

Die vorliegende Erfindung betrifft die gleichzeitige Halterung einer Mehrzahl von Behältern zur Aufbewahrung von Substanzen für medizinische, pharmazeutische oder kosmetische Anwendungen, insbesondere von Fläschchen (Vials), Spritzen (syringe) und Doppelkammer-Spritzen (dual-chamber syringe), Karpulen (cartridges), Doppelkammer-Karpulen (dual chamber cartridges) oder Vartridges, in einfacher und zuverlässiger Weise sowie dergestalt, dass diese, während diese in einer hierfür vorgesehenen Haltestruktur gehalten werden, in Abfüll- oder Bearbeitungsanlagen prozessiert oder weiter verarbeitet werden können, insbesondere in einem Steriltunnel, einer Abfüllanlage für flüssige medizinische oder pharmazeutische Anwendungen oder einem Gefriertrockenschrank hierfür. Ferner betrifft die vorliegende Erfindung einen Transport- und/oder Verpackungsbehälter mit zumindest einer solchen Haltestruktur sowie ein Verfahren zur Behandlung oder Verarbeitung solcher Behälter.

### Hintergrund der Erfindung

Als Behälter zur Aufbewahrung und Lagerung von medizinischen, pharmazeutischen oder kosmetischen Präparaten mit Verabreichung in flüssiger Form, insbesondere in vordosierten Mengen, werden in großem Umfang Medikamentenbehälter, wie beispielsweise Fläschchen, Ampullen oder Karpulen, eingesetzt. Diese weisen generell eine zylindrische Form auf, können aus Kunststoffen oder aus Glas hergestellt werden und sind kostengünstig in großen Mengen erhältlich. Für eine möglichst wirtschaftliche Befüllung der Behälter unter sterilen Bedingungen werden in zunehmendem Maße Konzepte eingesetzt, bei denen die Behälter gleich beim Hersteller der Behälter in Transport- und Verpackungsbehälter steril verpackt werden, die bei einem Pharmaunternehmen dann unter sterilen Bedingungen, insbesondere in einem sog. Steriltunnel, ausgepackt und dann weiter verarbeitet werden.

Zu diesem Zweck sind aus dem Stand der Technik div. Transport- und Verpackungsbehälter bekannt, in denen gleichzeitig eine Mehrzahl von Medikamentenbehältern in einer regelmäßigen Anordnung angeordnet sind. Dies hat Vorteile bei der automatisierten Weiterverarbeitung der Behälter, da die Behälter an kontrollierten Positionen und in vorgegebener Anordnung an Bearbeitungsstationen übergeben werden können, beispielsweise an Prozessautomaten, Roboter oder dergleichen. Hierzu werden Haltestrukturen eingesetzt, in welchen gleichzeitig eine Mehrzahl von Behältern in einer vorbestimmten regelmäßigen Anordnung gehalten werden können. Zur Übergabe an eine Bearbeitungsstation braucht einfach nur der Transport- und Verpackungsbehälter geeignet positioniert und geöffnet zu werden. Die nachgeordnete Bearbeitungsstation weiß dann, in welcher Position und Anordnung die weiter zu verarbeitenden Behälter angeordnet sind.

Ein solcher Transport- und Verpackungsbehälter und ein entsprechendes Verpackungskonzept sind beispielsweise in der US 8,118,167 B2 offenbart. Die Weiterverarbeitung der Behälter erfolgt jedoch stets in der Weise, dass die Haltestruktur, aus dem Transport- und Verpackungsbehälter entnommen wird, die Behälter aus der Haltestruktur, entnommen und vereinzelt werden und auf einer Fördereinrichtung, insbesondere einem Förderband, einzeln an die Bearbeitungsstationen übergeben und dort weiterverarbeitet werden. Dies begrenzt die erzielbare Geschwindigkeit bei der Weiterverarbeitung. Insbesondere bei der Vereinzelung der Behälter mit Hilfe von Zellenrädern oder dergleichen kommt es immer wieder dazu, dass einzelne Behälter unkontrolliert aneinanderstoßen, was zu einem unerwünschten Abrieb und in der Folge zu einer Verunreinigung des Behälterinnenraums oder der Prozessanlage sowie zu einer Beeinträchtigung des äußeren Erscheinungsbildes der Behälter führt, was unerwünscht ist.

US 8,100,263 B2 offenbart einen steril verpackbaren und transportierbaren Transport- und Verpackungsbehälter, in welchen eine plattenförmige Haltestruktur eingesetzt werden kann, in der eine Mehrzahl von Medikamentenbehältern in einer regelmäßigen Anordnung gehalten wird. Die Medikamentenbehälter können jedoch nicht in dem Transport- und Verpackungsbehälter oder in der Haltestruktur weiter verarbeitet werden, sondern müssen zunächst in der herkömmlichen Weise vereinzelt und an nachgeordnete Bearbeitungsstationen übergeben werden.

Ein weiterer vergleichbarer Transport- und Verpackungsbehälter und Haltestrukturen sind in der WO 2009/015862 A1 offenbart. Der Träger ist jedoch einteilig ausgebildet und weist keinen Rahmen auf, an dem der Träger in zwei entgegengesetzten Orientierungen abgestützt werden könnte.

Ein weiterer vergleichbarer Transport- und Verpackungsbehälter und eine Haltestrukturen sind in der WO 2011/135085 A1 offenbart. Zur Weiterverarbeitung müssen die Medikamentenbehälter jedoch stets vereinzelt werden. Eine chargenweise Weiterverarbeitung der Medikamentenbehälter, während diese in einer plattenförmigen Haltestruktur, wie vorstehend ausgeführt, aufgenommen sind, ist nicht möglich.

### Zusammenfassung der Erfindung

Aufgabe der vorliegenden Erfindung ist es, eine Haltestruktur zum gleichzeitigen Halten einer Mehrzahl von Behältern für medizinische, pharmazeutische oder kosmetische Anwendungen, dahingehend weiterzubilden, dass die Behälter in flexibler Anpassung an die Bedingungen bei deren Behandlung oder Verarbeitung einfach und zuverlässig gehalten sowie kostengünstig steril verpackt, wieder entpackt und weiterverarbeitet werden können. Gemäß einem weiteren Gesichtspunkt der vorliegenden Erfindung sollen ferner ein entsprechender Transport- und Verpackungsbehälter mit zumindest einer solchen Haltestruktur sowie ein entsprechendes Verfahren zur Behandlung oder Verarbeitung von Behältern bereitgestellt werden.

Diese Aufgaben werden gemäß der vorliegenden Erfindung durch eine Haltestruktur mit den Merkmalen nach Anspruch 1 bzw. Anspruch 2, durch einen Transport- und Verpackungsbehälter nach Anspruch 9 sowie durch ein Verfahren nach Anspruch 10 bzw. 11 gelöst. Weitere vorteilhafte Ausführungsformen sind Gegenstand der rückbezogenen Unteransprüche.

Somit wird eine Haltestruktur zum gleichzeitigen Halten einer Mehrzahl von Behältern einer vorbestimmten Länge bereitgestellt, die zur Aufbewahrung von Substanzen für medizinische, pharmazeutische oder kosmetische Anwendungen dienen oder diese enthalten, mit einem Träger, der eine Mehrzahl von Haltemitteln aufweist und ausgelegt ist, um die Behälter wahlweise in einer ersten Orientierung (beispielsweise aufrecht) oder in einer zweiten Orientierung, die entgegengesetzt zur ersten Orientierung ist (also beispielsweise kopfüber), zu halten.

Gemäß einer ersten Ausführungsform ist die Haltestruktur erfindungsgemäß zweiteilig ausgebildet und umfasst den Träger und einen Rahmen, an welchem der Träger in einer ersten vorbestimmten Orientierung oder in einer zweiten vorbestimmten Orientierung, die entgegengesetzt zu der ersten vorbestimmten Orientierung ist, abstützbar ist, wobei der Träger so auf die axiale Länge der Behälter abgestimmt, dass die oberen Enden der Behälter in einer ersten Stellung, in welcher die Behälter in der ersten Orientierung an dem Träger gehalten sind und der Träger in der ersten vorbestimmten Orientierung an dem Rahmen abgestützt ist, unter dem gleichen Abstand zum oberen Rand des zur Abstützung des Trägers dienenden Rahmens angeordnet sind wie die unteren Enden der Behälter in einer zweiten Stellung, in welcher die Behälter in der zweiten Orientierung an dem Träger gehalten sind und der Träger in der zweiten vorbestimmten Orientierung an dem Rahmen abgestützt ist, und die oberen Enden und/oder unteren Enden der Behälter, während diese an dem Träger gehalten sind, für eine Weiterverarbeitung der Behälter zugänglich sind.

Gemäß einer alternativen zweiten Ausführungsform ist die Haltestruktur erfindungsgemäß zweiteilig ausgebildet und umfasst den Träger und einen Rahmen, an welchem der Träger in einer ersten Orientierung oder in einer zweiten Orientierung, die entgegengesetzt zu der ersten Orientierung ist, abstützbar ist, wobei der Träger so auf die Länge der Behälter abgestimmt ist, dass die oberen Enden der Behälter in einer ersten Stellung, in welcher die Behälter in einer vorbestimmten Orientierung an dem Träger gehalten sind und der Träger in der ersten Orientierung an dem Rahmen abgestützt ist, unter dem gleichen Abstand zum oberen Rand des zur Abstützung des Trägers dienenden Rahmens angeordnet sind wie die oberen Enden der Behälter in einer zweiten Stellung, in welcher die Behälter in der gleichen vorbestimmten Orientierung an dem Träger gehalten sind und der Träger in der zweiten Orientierung an dem Rahmen abgestützt ist, wobei die oberen Enden der Behälter, während der Träger in der ersten Orientierung an dem Rahmen abgestützt ist und die Behälter in der vorbestimmten Orientierung an dem Träger gehalten sind, für eine Weiterverarbeitung der Behälter zugänglich sind, und die oberen Enden und/oder unteren Enden der Behälter, während der Träger in der zweiten Orientierung an dem Rahmen abgestützt ist und die Behälter in der vorbestimmten Orientierung an dem Träger gehalten sind, für eine Weiterverarbeitung der Behälter zugänglich sind

Somit brauchen Prozessstationen, in denen die Behälter weiter behandelt oder verarbeitet werden, unabhängig davon, ob die Behälter an der Haltestruktur in der ersten Orientierung (also beispielsweise aufrecht stehend) oder in der zweiten Orientierung (also beispielsweise kopfüber) gehalten werden, in ihrer Höhe nicht verstellt werden, um die Behälter zu behandeln oder zu verarbeiten. Denn die oberen und unteren Enden der Behälter befinden sich in beiden Stellungen auf dem gleichen Höhenniveau. Dies erleichtert die Behandlung und Verarbeitung der Behälter erfindungsgemäß erheblich, da der Aufwand hinsichtlich Justierung, Steuerung und Automatisierung erheblich vereinfacht werden kann.

Mit dem Begriff "unter dem gleichen Abstand" sollen im Sinne der vorliegenden Anmeldung natürlich zunächst unvermeidliche Längentoleranzen der Behälter mit berücksichtigt sein. Anderseits sollen gemäß weiteren Ausführungsformen auch Toleranzen mit berücksichtigt sein, die durch eine unterschiedliche Halterung der oberen und unteren Enden der Behälter an dem Träger bedingt sind und bevorzugt maximal von der Größenordnung der axialen Länge eines verengten oberen Hals- oder Nackenabschnitts der zu haltenden Behälter sind oder die diese axiale Länge, im Vergleich zur Gesamtlänge der zu haltenden Behälter, allenfalls geringfügig überschreiten, beispielsweise um maximal 20%, bevorzugter um maximal 10% der axialen Länge des verengten oberen Hals- oder Nackenabschnitts.

Gemäß einer weiteren Ausführungsform weist der Träger eine Mehrzahl von Öffnungen auf, denen jeweils bevorzugt zwei Haltemittel zugeordnet sind, um die Behälter zu halten, insbesondere mittels eines Form- oder Reibschlusses. Dabei werden die Behälter bevorzugt so von den Haltemitteln an dem Träger gehalten, dass diese sich durch die Öffnungen der Trägers hindurch erstrecken. Dabei können die oberen Enden der Behälter über einen oberen Rand des Trägers vorstehen und/oder die unteren Enden der Behälter über einen unteren Rand des Trägers vorstehen, sodass diese für eine Weiterverarbeitung der Behälter noch besser zugänglich sind.

Gemäß einer weiteren Ausführungsform sind die Behälter in der ersten und zweiten Orientierung an dem Träger abgestützt oder an dem Träger axial gesichert gehalten, sodass diese auch bei einer Umkehrung der Orientierung (Wenden) des Trägers sicher an diesem gehalten sind.

Gemäß einer weiteren Ausführungsform sind an dem Träger, der bevorzugt flächig und insbesondere rechteckförmig ausgebildet ist, als Haltemittel an jeder Öffnung bevorzugt zwei Haltezungen vorgesehen, die am Rand einer jeweiligen Öffnung vorgesehen sind und von einer Oberseite des Trägers abragen, um den jeweiligen Behälter in der Öffnung zu halten. Dabei sind die Haltezungen so ausgelegt, dass diese beim Einführen der Behälter in die Öffnungen elastisch weggeschwenkt oder weggeklappt werden, und ferner so auf die Behälter abgestimmt, dass die Behälter mit radialem Spiel von den Haltezungen gehalten sind. Das radiale Spiel ermöglicht, dass Behälter mit unterschiedlichen radialen Toleranzen und/oder Außenabmessungen von demselben Träger zuverlässig gehalten werden können. Das radiale Spiel ist zweckmäßig so ausgelegt und auf die Außenkontur und -abmessung der Behälter abgestimmt, dass niemals gleichzeitig sämtliche Haltezungen den verengten Halsabschnitt am oberen Ende der Behälter, insbesondere Fläschchen, berühren. Gleichzeitig verhindert das radiale Spiel auch ein unerwünschtes Verspannen oder gar Aufwölben des Trägers beim Halten von Behältern mit unterschiedlichen radialen Toleranzen und/oder Außenabmessungen, was erhebliche Vorteile insbesondere bei der gleichzeitigen Prozessierung einer Mehrzahl von Behältern bietet, während diese von dem Träger gehalten sind, beispielsweise bei der Gefriertrocknung bei Prozessierung bei sehr niedrigen Temperaturen.

Die Haltezungen sind dabei gemäß einer weiteren Ausführungsform ausreichend elastisch ausgebildet oder gelagert, sodass die Behälter axial, d.h. in Richtung der Längsachse der Behälter und senkrecht zur Ebene des Trägers, von der Oberseite oder Unterseite des Trägers her in die Öffnungen oder Aufnahmen eingeschoben werden können, insbesondere unter elastischer Verformung der Haltezungen, beispielsweise unter Wegbiegen derselben. Die Bestückung des Trägers mit Behältern kann somit einfach automatisiert werden, was durch eine regelmäßige Anordnung der Öffnungen oder Aufnahmen noch weiter begünstigt wird, bevorzugt durch eine regelmäßige Anordnung der Öffnungen oder Aufnahmen in einer zweidimensionalen Matrix.

Als bevorzugte Stelle, an der die Behälter an den Haltezungen gehalten oder abgestützt sind, hat sich die Unterseite eines verbreiterten oberen Randabschnittes der Behälter erwiesen, wie dieser insbesondere bei Fläschchen (vials) typischerweise als sog. Rollrand oder Schulter vorgesehen ist. In diesem Bereich steht eine Abstütz- oder Lagerfläche zum Halten oder Abstützen der Behälter mit einer ausreichenden Erstreckung in Radialrichtung der Öffnungen oder Aufnahmen zur Verfügung, um das vorgenannte radiale Spiel bei der Halterung der Behälter ohne Weiteres zu realisieren.

Weil die Behälter in den Öffnungen oder Aufnahmen des Trägers mit sehr geringem Kraftaufwand angehoben oder bewegt werden können, diese beispielsweise gedreht werden können, können die Behälter, während diese sich in dem Träger befinden und von diesem gehalten oder zumindest gerührt werden, erfindungsgemäß ohne Weiteres bearbeitet werden. Als besonders vorteilhaft hat sich diese Art der Halterung z.B. beim Verschließen der Behälter durch Bördeln eines Metalldeckels erwiesen. Die hierzu erforderlichen Vorgänge können an dem Metalldeckel ausgeführt werden, während die Behältern in den Öffnungen oder Aufnahmen des Trägers gehalten oder zumindest geführt sind. Als besonders vorteilhaft hat sich diese Art der Halterung auch bei der Prozessierung von Behältern erwiesen, während die Behälter in der Haltestruktur gehalten bzw. aufgenommen sind. Beispielsweise können die Träger mit den darin aufgenommenen bzw. gehaltenen Behältern in einen Gefriertrockner oder Gefriertrockenschrank eingebracht werden. Aufgrund der Halterung der Behälter mit einem gewissen Spiel in den Trägem kann gewährleistet werden, dass die Böden von sämtlichen Behältern auf einer kühlenden Unterlage gleichmäßig aufliegen, beispielsweise auf einem Kühlfinger des Gefriertrockners oder Gefriertrockenschranks. Oder die Behälter können ohne größeren Kraftaufwand in den Öffnungen oder Aufnahmen des Trägers angehoben und zur Prozessierung gehandhabt werden.

Gemäß einer bevorzugten Ausführungsform sind die Haltezungen als elastische Haltezungen ausgebildet, verfügen diese jedoch über eine ausreichende Elastizität, um beim Einführen der Behälter in die Öffnungen oder Aufnahmen ausreichend elastisch weggeschwenkt oder weggeklappt zu werden, um den Behältern den Weg in die Öffnungen oder Aufnahmen freizugeben. Dies lässt sich durch geeignete Dimensionierung, Materialwahl und Auslegung der Materialstärke der Haltezungen ohne Weiteres erreichen. Bevorzugt sind die Haltezungen somit aus einem Kunststoff ausgebildet.

Gemäß einer Ausführungsform sind die Haltezungen elastisch gegen eine Haltestellung vorgespannt, bevorzugt mittels eines elastischen Rückstellelements, beispielsweise einer Rückstellfeder oder eines Kunststoffblättchens oder elastischen Kunststoffgebildes, das mit der zugeordneten Haltezunge geeignet zusammenwirkt und auf der Oberseite des Trägers vorgesehen oder ausgebildet ist.

Gemäß einer Ausführungsform sind die Haltezungen so auf die Behälter abgestimmt, dass die Behälter mit einem verbreiterten Rand, der an einem oberen Ende der Behälter ausgebildet ist, also insbesondere mit dem vorgenannten Rollrand, lose auf Oberseiten der Haltezungen aufliegen. Die Behälter können somit ohne größeren Widerstand wieder aus den Öffnungen oder Aufnahmen nach oben entnommen werden.

Gemäß einer Ausführungsform umgreifen die Haltezungen den verbreiterten Rand dergestalt, dass die Behälter mit radialem Spiel oder mit radialem und axialem Spiel von den Haltezungen gehalten sind. Auf diese Weise können die Behälter in den Öffnungen axial verliersicher gehalten werden, sodass der Träger problemlos gewendet werden kann. Zum Entnehmen der Behälter aus den Öffnungen brauchen die Haltezungen nur wiederum in der Art, wie beim Einführen der Behälter, zurückgeschwenkt oder zurückgeklappt werden.

Gemäß einer weiteren Ausführungsform sind die Öffnungen auf einer Seite des Trägers, also auf der Oberseite oder der Unterseite des Trägers, zumindest abschnittsweise von einer Seitenwand oder einem oder mehreren Zapfen begrenzt, um eine Berührung von Behältern in unmittelbar benachbarten Öffnungen oder Aufnahmen zu verhindern. Dabei sind die Seitenwände oder Zapfen ganz besonders bevorzugt so ausgebildet, dass die Behälter von dieser Seite des Trägers her frei zugänglich sind, also von der Oberseite oder von der Unterseite des Trägers. Dabei können die Seitenwände von benachbarten Öffnungen oder Aufnahmen auch miteinander verbunden sein, was vorteilhaft zu einer weiteren Versteifung des Trägers beiträgt. Bevorzugt sind die Seitenwände einstückig mit dem Träger ausgebildet, was beispielsweise in Kunststoff-Spritzgusstechnik einfach realisiert werden kann.

Die Böden bzw. unteren Enden der in den Öffnungen aufgenommenen Behälter stehen bevorzugt von den Enden der Seitenwände vor, sodass die Böden bzw. unteren Enden der Behälter von der Unterseite oder der Oberseite des Trägers her frei zugänglich sind. Dies ermöglicht, dass die Behälter prozessiert werden können, während diese an dem Träger in den Öffnungen oder Aufnahmen gehalten sind, wie weiter unten ausgeführt.

Gemäß einer weiteren Ausführungsform können an dem Rahmen und dem Träger jeweils formschlüssig miteinander zusammen wirkende Elemente vorgesehen sein, um in der jeweiligen Orientierung eine unterschiedliche Höhendifferenz zwischen dem Rahmen und dem Träger festzulegen.

Ein weiterer Gesichtspunkt der vorliegenden Erfindung betrifft ferner einen Transport- und Verpackungsbehälter mit zumindest einer Haltestruktur, wie vorstehend ausgeführt und nachfolgend weiter im Detail offenbart. Dabei stehen die Behälter jeweils nicht über einen oberen Rand und/oder unteren Rand des Transport- und Verpackungsbehälters vor, wenn die Behälter an dem Träger in der ersten Stellung oder in der zweiten Stellung gehalten sind, sodass der Transport- und Verpackungsbehälter zum Transport der Behälter steril versiegelt werden kann, beispielswiese mittels einer Versiegelungsfolie, die auf den oberen Rand und/oder auf den unteren Rand des Transport- und Verpackungsbehälters aufgeklebt wird.

Ein weiterer Gesichtspunkt der vorliegenden Erfindung, wie mit Anspruch 10 bzw. Anspruch 11 beansprucht, betrifft ferner ein Verfahren zur Behandlung oder Verarbeitung von Behältern, die zur Aufbewahrung von Substanzen für medizinische, pharmazeutische oder kosmetische Anwendungen dienen oder diese enthalten, wobei die Behälter an zumindest einem Ende offen ausgebildet sind, unter Verwendung einer Haltestruktur, wie hierin offenbart, und/oder eines Transport- und Verpackungsbehälters, wie hierin offenbart.

Bei der Behandlung oder Verarbeitung der Behälter kann es sich insbesondere um ein Reinigen der Behälter und/oder ein Wiegen der Behälter und/oder ein Befüllen der Behälter und/oder ein Verschließen der Behälter mit Stopfen oder Kappen und/oder ein Bördeln von Metallkappen auf die Behälter handeln.

Nach einer weiteren Ausführungsform des Verfahrens kann die Behandlung oder Verarbeitung der Behälter einen Schritt zur Messung einer Füllmenge der Behälter umfassen, bei dem die Behälter so an dem Träger gehalten sind, dass ein Laserstrahl jeweils einen verengten Halsabschnitt der Behälter durchstrahlt und der Laserstrahl mit Hilfe eines Sensors detektiert wird, wobei auf Grundlage eines Messsignals des Sensors auf die jeweilige Füllmenge der Behälter geschlossen wird, insbesondere auf einen Füllstand der Behälter.

Nach einer weiteren Ausführungsform des Verfahrens wird bei dem Verfahren ein Behälter mit einem Stopfen verschlossen, mit den folgenden Schritten: Anordnen eines Dichtungselements auf einem Ende des Behälters, um das Ende des Behälters gegen die Umgebung abzudichten; Einführen des Stopfens in einem komprimierten Zustand mittels einer Greifeinrichtung, insbesondere eines Greifers, in das Ende des Behälters; Anlegen eines Unterdrucks an das Ende des Behälters über einen Ringspalt zwischen dem Greifer und einer Innenseite des Behälters; und Entspannen des Stopfens, bis der Stopfen an der Innenseite des Behälters anliegt, um das Ende des Behälters gegen die Umgebung abzudichten. Weil der Stopfen in einem komprimierten Zustand in des offene Ende des Behälters eingeführt wird und das Innenvolumen des Behälters mittels des Dichtungselements gegen die Umgebung abgedichtet ist, kann ein geeigneter Unterdruck an das Innenvolumen des Behälters in einfacher Weise angelegt werden, um Luft oder Gase aus dem Innenvolumen abzusaugen, bevor der Stopfen dann das Ende verschließt.

Nach einer weiteren Ausführungsform des Verfahrens umfasst der Greifer einen Zylinder, dessen Außendurchmesser kleiner ist als der Innendurchmesser am Ende des Behälters, wobei das Einführen des Stopfens in einem komprimierten Zustand mittels des Greifers in das Ende des Behälters weiterhin umfasst: Komprimieren des Stopfens durch Einführen des Stopfens in den Zylinder; Verstellen des Zylinders gemeinsam mit dem Vakuum-Greifer, um den Stopfen in das Ende des Behälters einzuführen; Drücken des Dichtungselements, um das Ende des Behälters gegen die Umgebung abzudichten; und Herausschieben des Stopfens aus dem Zylinder weiter in das Ende des Behälters hinein und hin zur Oberfläche der Flüssigkeit, die in dem Behälter aufbewahrt ist. Der Zylinder wirkt dabei als Begrenzungs- und Führungsmittel für den Stopfen und den Greifer, um einen Ringspalt zwischen der Außenwand des Zylinders und der Innenwand des Behälters zuverlässig zu begrenzen.

Nach einer weiteren Ausführungsform des Verfahrens ist das Dichtungselement der Boden eines Trägers oder einer Haltestruktur, wie vorstehend ausgeführt, wobei die Behälter so auf dem Boden des Trägers oder der Haltestruktur angeordnet werden, dass die Enden der Behälter mit Öffnungen, die in dem Boden ausgebildet sind, fluchten und dass die Stopfen durch die Öffnungen hindurch in die Enden der Behälter eingeführt werden. Dabei kann der Stopfen in dem Zylinder beim Einführen in die Öffnungen komprimiert aufgenommen sein. Somit können die Stopfen insbesondere dann gesetzt werden, wenn die Behälter in ihrer üblichen Transportstellung in einem Träger, wie hierin offenbart, ruhen, beispielsweise wenn der Träger aus einem Transport- und Verpackungsbehälter herausgenommen wird, wie hierin offenbart. Die Stopfen können somit erfindungsgemäß in die offenen Enden von Behältern eingesetzt werden, während diese in einem Träger (Nest) gehalten sind. Bei den Behältern kann es sich dabei insbesondere um Zylinderampullen oder Karpulen handeln, die in einem Träger gehalten werden, der in einem Transport- und Verpackungsbehälter steril aufbewahrt und transportiert wird, wobei der Transport- und Verpackungsbehälter bei einem Pharmahersteller geöffnet wird und die Zylinderampullen oder Karpulen dann befüllt und durch Setzen eines Stopfen in der hierin offenbarten Weise verschlossen werden, während die Zylinderampullen oder Karpulen in dem Träger gehalten sind.

Das Dichtungselement kann bei diesem Verfahren auch als separates Bauteil zugeführt werden, insbesondere auch in Gestalt einer Platte mit mehreren ringförmigen Dichtungselementen, die über Stege oder dergleichen miteinander verbunden sind.

### Figurenübersicht

Nachfolgend wird die Erfindung in beispielhafter Weise und unter Bezugnahme auf die beigefügten Zeichnungen beschrieben werden, woraus sich weitere Merkmale, Vorteile und zu lösende Aufgaben ergeben werden. Es zeigen:
- Fig. 1: einen Behälter zur Aufbewahrung von Substanzen für medizinische, pharmazeutische oder kosmetische Anwendungen, der als Fläschchen (vial) ausgebildet ist;
- Fig. 2a bis 2g: die Grundbestandteile einer zweiteilig ausgebildeten Haltestruktur gemäß einer ersten Ausführungsform der vorliegenden Erfindung;
- Fig. 3a bis 3g: die Aufnahme einer solchen Haltestruktur in einem Transport- und Verpackungsbehälter gemäß der vorliegenden Erfindung;
- Fig. 3h und 3i: in einer Gegenüberstellung die Haltestruktur gemäß den Figuren 2a-2g in einer ersten Stellung und in einer zweiten Stellung der Behälter;
- Fig. 4a bis 4j: eine Haltestruktur sowie einen Transport- und Verpackungsbehälter gemäß einer weiteren Ausführungsform der vorliegenden Erfindung;
- Fig. 4k: in einer perspektivischen Explosionsansicht den Transport- und Verpackungsbehälter gemäß den Figuren 3a bis 3g mit einer Haltestruktur gemäß den Figuren 2a bis 2g;
- Fig. 41: in einer schematischen Darstellung ein Verfahren zur gleichzeitigen Behandlung oder Verarbeitung einer Mehrzahl von Behältern gemäß der vorliegenden Erfindung;

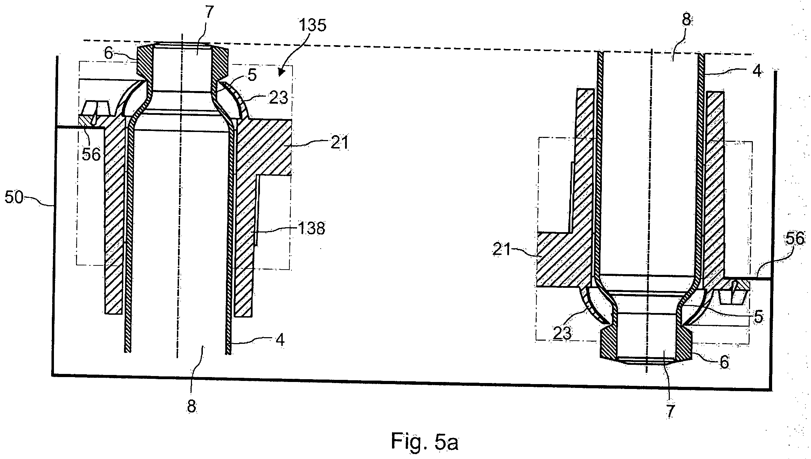

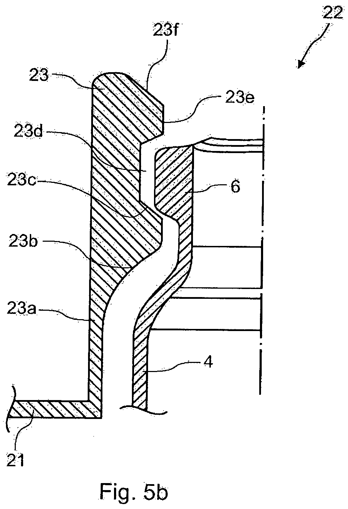

- Fig. 5a und 5b: weitere Beispiele für die Halterung eines Behälters an einem Träger einer Haltestruktur, gemäß der vorliegenden Erfindung;

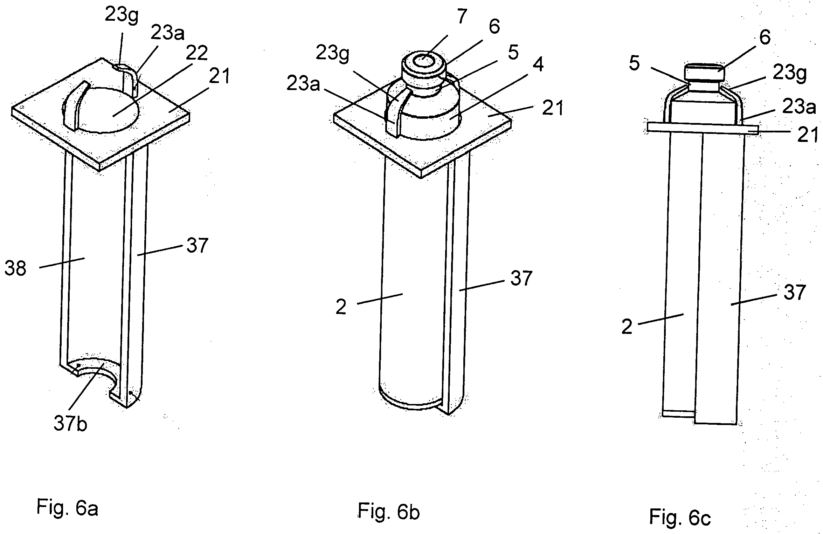

- Fig. 6a bis 6h: weitere Beispiele für die Halterung eines Behälters an einem Träger einer Haltestruktur gemäß der vorliegenden Erfindung;

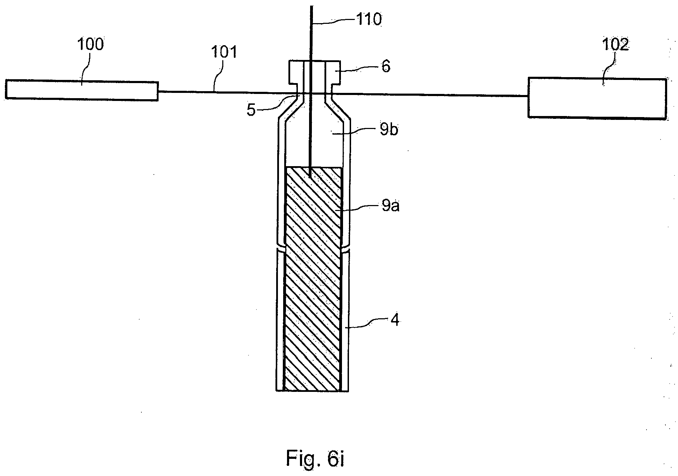

- Fig. 6i: einen Prozessschritt bei einem Verfahren gemäß der vorliegenden Erfindung zur Ermittlung der Füllmenge oder des Füllstands eines Behälters mit Hilfe eines Lasers;

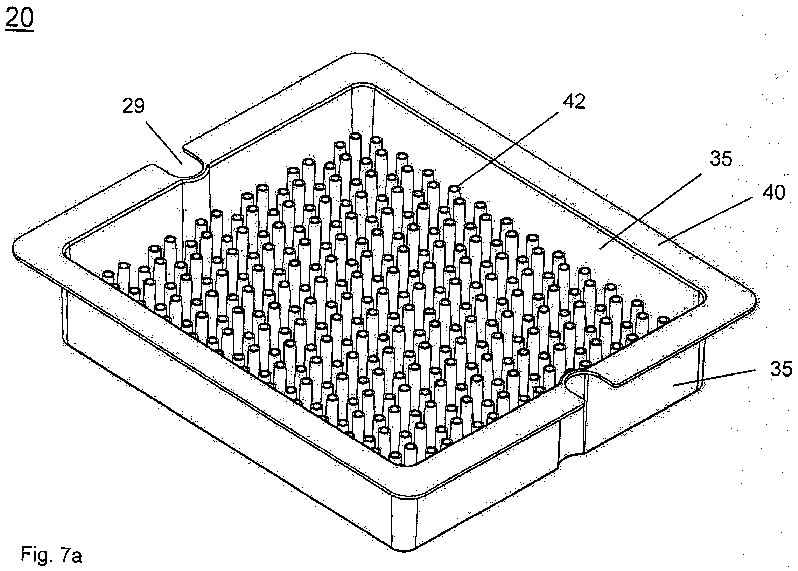

- Fig. 7a bis 7e: eine Haltestruktur gemäß einer weiteren Ausführungsform, die einem besseren Verständnis der vorliegenden Erfindung dienen soll;

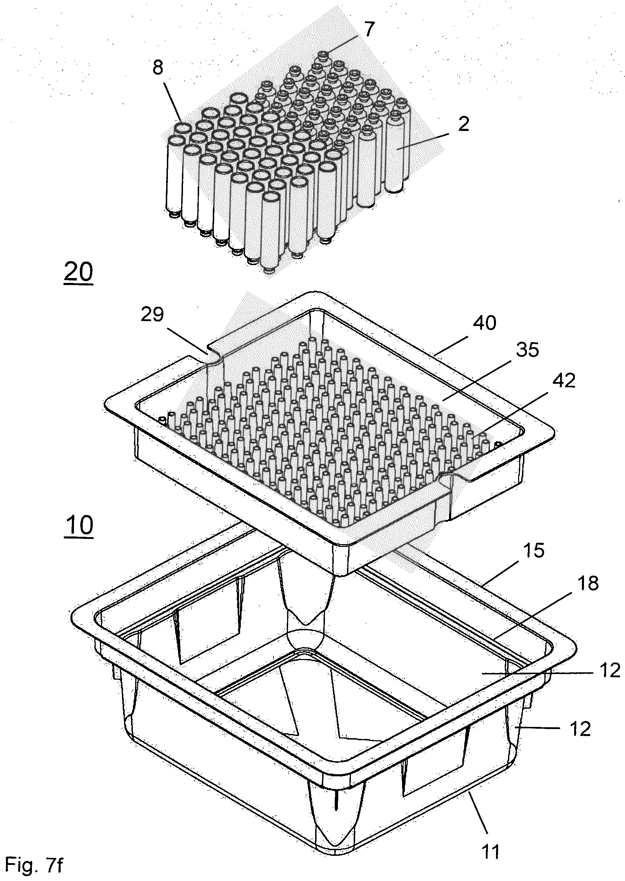

- Fig. 7f: in einer perspektivischen Explosionsansicht einen Transport- und Verpackungsbehälter mit der Haltestruktur gemäß den Figuren 7a bis 7e;

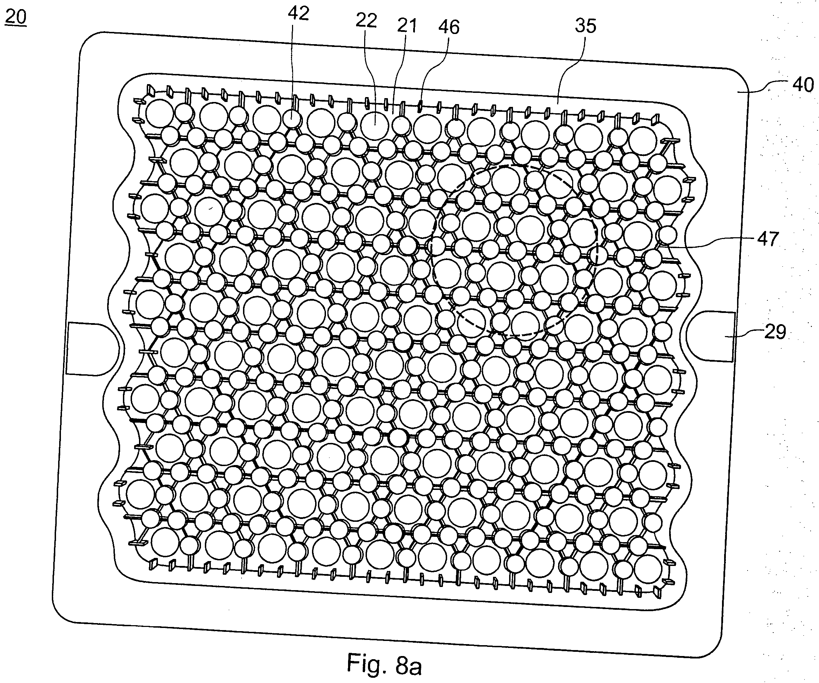

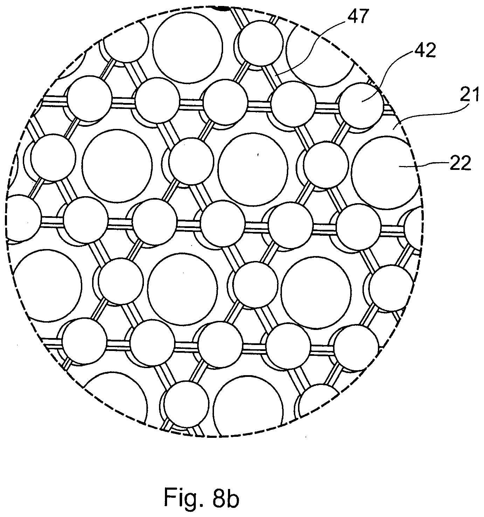

- Fig. 8a und 8b: in einer Draufsicht und einer stark vergrößerten Teilansicht eine Haltestruktur gemäß einer weiteren Ausführungsform der vorliegenden Erfindung;

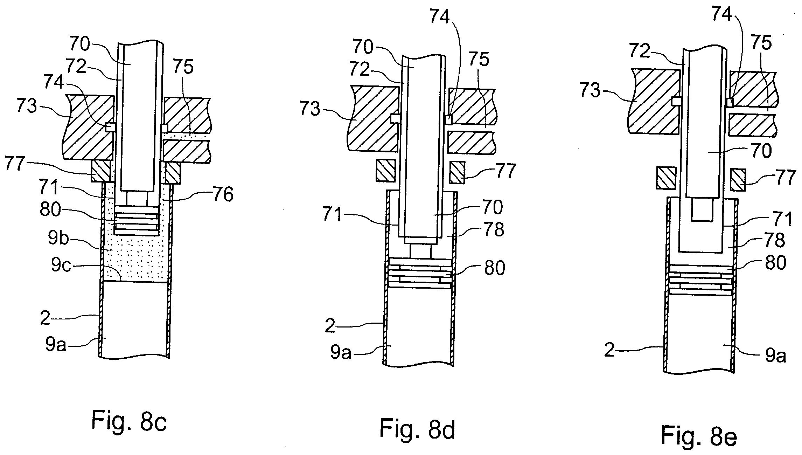

- Fig. 8c bis 8e: in schematischen Teilschnitten drei unterschiedlichen Phasen eines Prozessschritts zum Setzen von Stopfen in Karpulen bei einem weiteren Verfahren gemäß der vorliegenden Erfindung; und

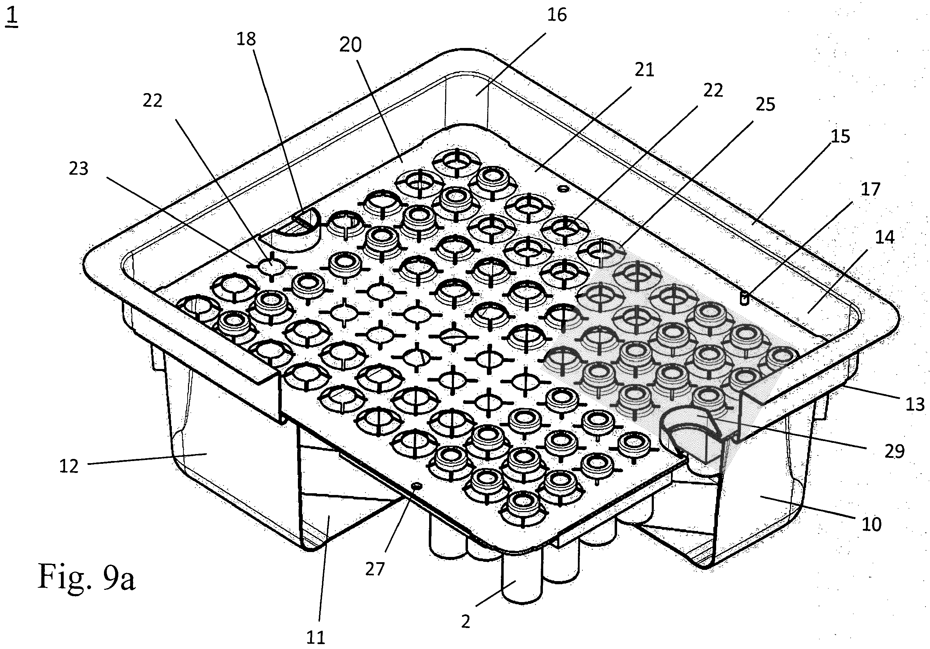

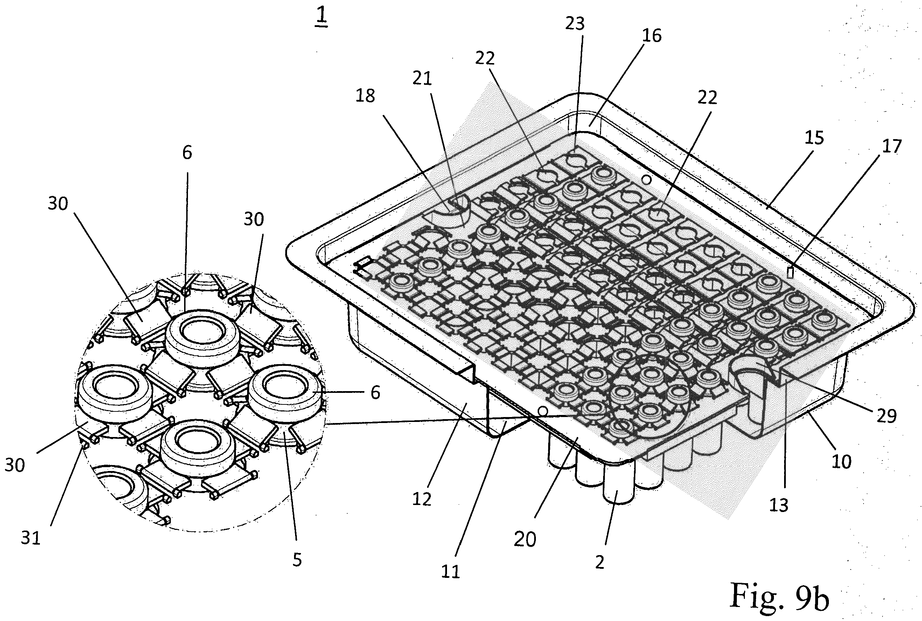

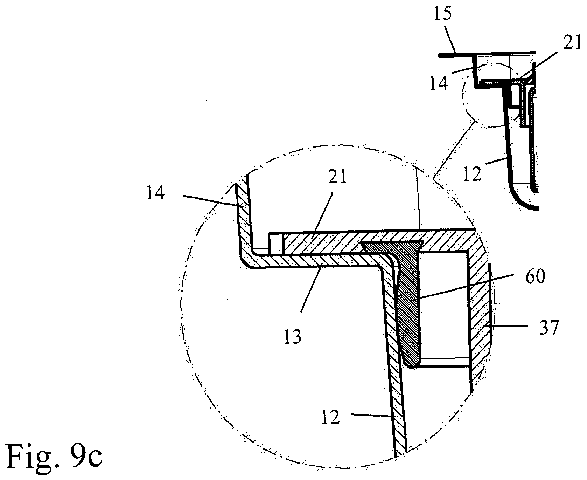

- Fig. 9a: in einem perspektivischen Teilschnitt einen Transport- und Verpackungsbehälter zur Verwendung mit einer erfindungsgemäßen Haltestruktur, wobei in der Fig. 9a die Haltestruktur einteilig ausgebildet dargestellt ist.

In den Figuren bezeichnen identische Bezugszeichen identische oder im Wesentlichen gleichwirkende Elemente oder Elementgruppen.

### Ausführliche Beschreibung von bevorzugten Ausführungsbeispielen

Eine Haltestruktur sowie ein Transport- und Verpackungsbehälter, der eine solche Haltestruktur aufnimmt, dienen gemäß der vorliegenden Erfindung, wie nachfolgend beschrieben, der gleichzeitigen Halterung einer Mehrzahl von Behältern zur Aufbewahrung von Substanzen für medizinische, pharmazeutische oder kosmetische Anwendungen, und zwar bevorzugt in einer regelmäßigen Anordnung, insbesondere in einer Matrixanordnung unter regelmäßigen Abständen der Behälter zueinander, entlang von zwei unterschiedlichen Raumrichtungen, bevorzugt entlang von zwei zueinander orthogonalen Raumrichtungen oder in regelmäßigen Reihen, die relativ zueinander versetzt angeordnet sind.

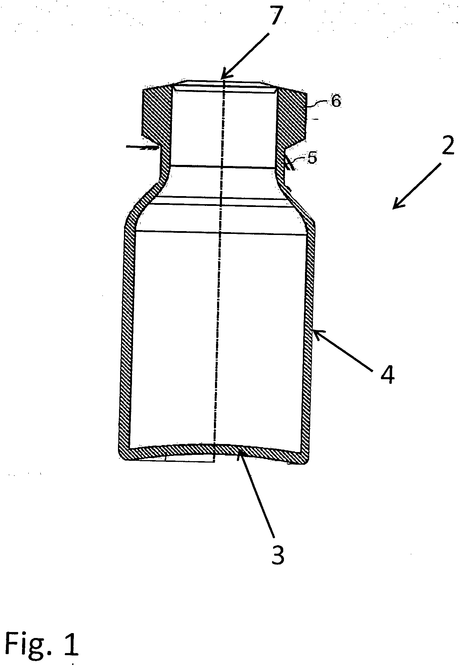

Ein Beispiel für derartige Medikamentenbehälter in Gestalt von Fläschchen (vials) ist in der Fig. 1 schematisch in einem Längsschnitt dargestellt. Diese weisen eine zylindrische Grundform auf, mit einer zylinderförmigen Seitenwand mit - im Rahmen der Toleranzen - konstantem Innen- und Außendurchmesser, die von einem flach ausgebildeten Flaschenboden 3 senkrecht abragt und nahe dem oberen offenen Ende des Fläschchens in einen verengten Halsabschnitt 5 von vergleichsweise geringer axialer Länge und anschließend in einen verbreiterten oberen Rand 6 (auch als Rollrand bezeichnet) übergeht, der einen größeren Außendurchmesser aufweist als der zugeordnete Halsabschnitt 5 und zur Verbindung mit einem Verschlusselement ausgelegt ist. Wie man der Fig. 1 entnehmen kann, ist die Unterseite des Rollrands 6 abgeschrägt ausgebildet und erstreckt sich unter einem spitzen Winkel abwärts und hin zu dem verengten Halsabschnitt 5. Gemäß weiteren Ausführungsformen kann die Unterseite des Rollrands 6 auch flach ausgebildet sein und sich im Wesentlichen senkrecht zu dem verengten Halsabschnitt 5 radial erstrecken.

Der Halsabschnitt 5 kann glattwandig ohne Außengewinde ausgebildet sein oder kann mit einem Außengewinde zum Aufschrauben eines Verschlusselements versehen sein. Beispielsweise kann in die Innenbohrung des Halsabschnitts 5 und des oberen Rands 6 ein Stopfen (nicht dargestellt) eingeführt werden, dessen oberes Ende mit dem oberen Rand 6 des Fläschchens gasdicht und geschützt gegen das Eindringen von Verunreinigungen in das Fläschchen mit dem oberen Rand 6 verbunden ist, beispielsweise durch Crimpen oder Bördeln einer nicht dargestellten Metallschutzfolie. Derartige Fläschchen sind radial symmetrisch und aus einem durchsichtigen oder eingefärbten Glas oder auch durch Blasformen oder Kunststoff-Spritzgusstechniken aus einem geeigneten Kunststoffmaterial ausgebildet, und können grundsätzlich innenbeschichtet sein, so dass das Material des Fläschchens möglichst wenig Verunreinigungen an die aufzunehmende Substanz abgibt.

Ein weiteres Beispiel für Behälter im Sinne der vorliegenden Anmeldung sind Ampullen, Karpulen oder Spritzen- oder Injektionsbehältnisse.

Im Sinne der vorliegenden Erfindung dienen derartige Behälter (container) zur Aufbewahrung von Substanzen oder Wirkstoffen für medizinische, pharmazeutische oder kosmetische Anwendungen, die in einer oder auch mehreren Komponenten in fester oder flüssiger Form in dem Behälter aufbewahrt werden sollen. Während nachfolgend Behälter offenbart werden, die zylindrisch sind, sei daraufhingewiesen, dass die Behälter im Sinne der vorliegenden Erfindung auch ein anderes Profil haben können, beispielsweise ein quadratisches, rechteckförmiges oder vieleckiges Profil.

Unweigerlich weisen solche Behälter herstellungsbedingt Toleranzen auf, die gerade bei Glasbehältern einen oder mehrere Zehntel Millimeter betragen können. Um solche Fertigungstoleranzen kompensieren zu können und gleichzeitig zu gewährleisten, dass sämtliche Flaschenböden 3 in einer Ebene angeordnet werden können, werden die Behälter erfindungsgemäß an einer Halterungsstruktur fixiert. Die Halterung der Behälter wird dabei im Übergangsbereich des verengten Halsabscbnitts 5 zum verbreiterten oberen Rand 6 realisiert. Insbesondere kann die Unterseite des Rands 6 der Behälter im Übergangsbereich zum verengten Halsabschnitt 5 auf den oberen Enden von Haltezungen aufliegen, wie nachfolgend anhand der Fig. 5a näher beschrieben.

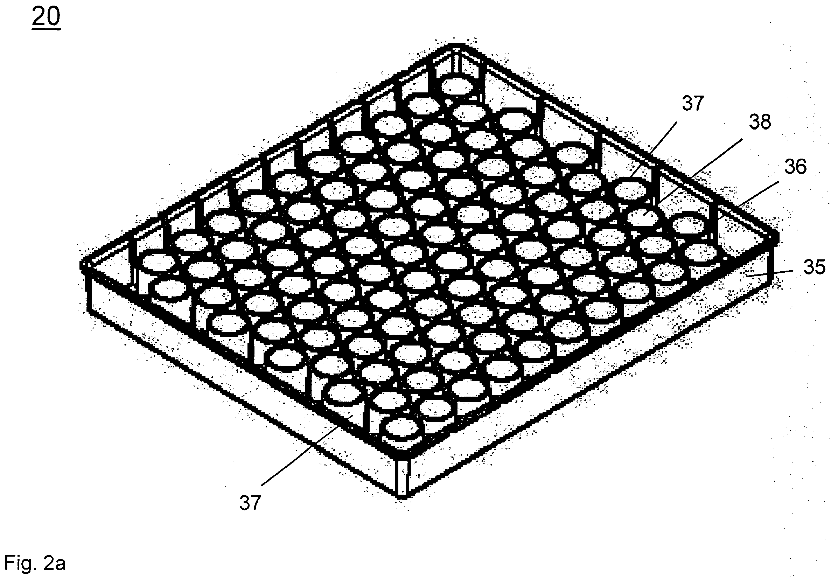

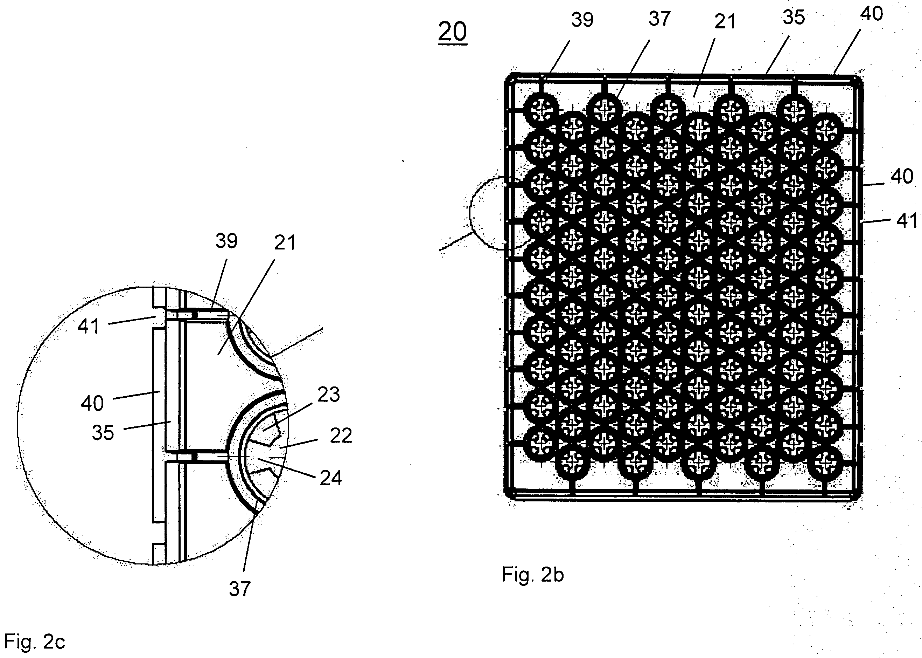

Zum gleichzeitigen Halten einer Mehrzahl von Behältern wird gemäß einer ersten Ausführungsform der vorliegenden Erfindung, wie in den Fig. 2a bis 2g dargestellt, ein flächiger rechteckförmiger Träger 20 (in dieser Anmeldung auch als Haltestruktur bezeichnet) bereitgestellt, der aus einem Kunststoff ausgebildet ist, beispielsweise ausgestanzt oder spritzgegossen ist, und der eine Mehrzahl von Öffnungen 22 zur Aufnahme der Glasfläschchen 2 aufweist (vgl. Fig. 2c). Die Öffnungen 22 sind in einer regelmäßigen zweidimensionalen Anordnung angeordnet, bei dem dargestellten Ausführungsbeispiel in einer Matrix-Anordnung aus Reihen und sich rechtwinklig dazu erstreckenden Spalten, die unter gleichen Abständen zueinander und regemäßig zueinander versetzt in einer wiederkehrenden Anordnung angeordnet sind.

Die Öffnungen 22 sind von zylindrischen Seitenwänden 37 (vgl. Fig. 2b) auf der Oberseite des Trägers 20 umgeben, die bevorzugt umlaufend ausgebildet sind, jedoch auch als nur vergleichsweise kurze Seitenwandabschnitte ausgebildet sein können, um eine zugeordnete Öffnung 22 nur abschnittsweise zu begrenzen. In jedem Fall wird eine Kollision von Behältern, die in unmittelbar benachbarten Öffnungen 22 aufgenommen sind, durch die Seitenwände 37 verhindert.

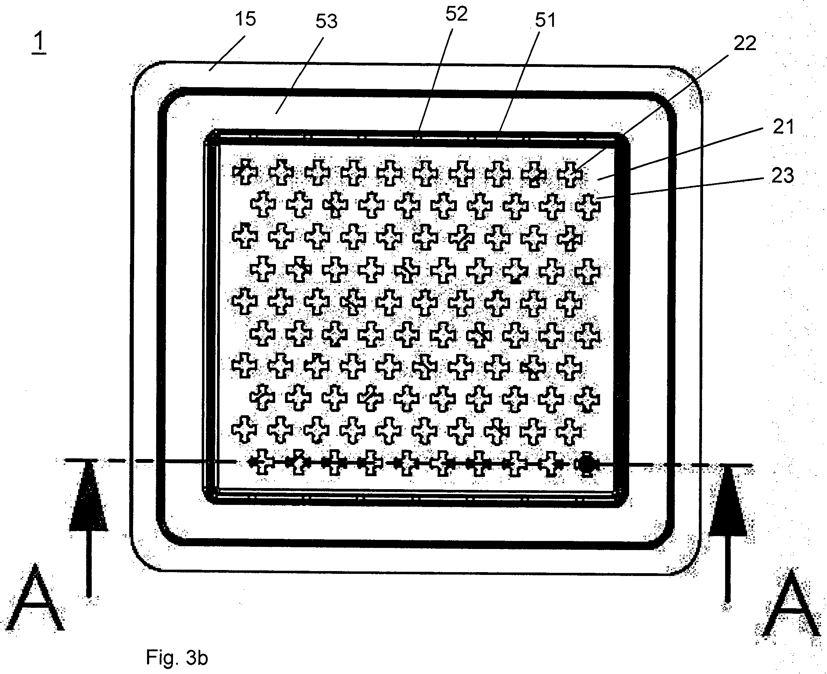

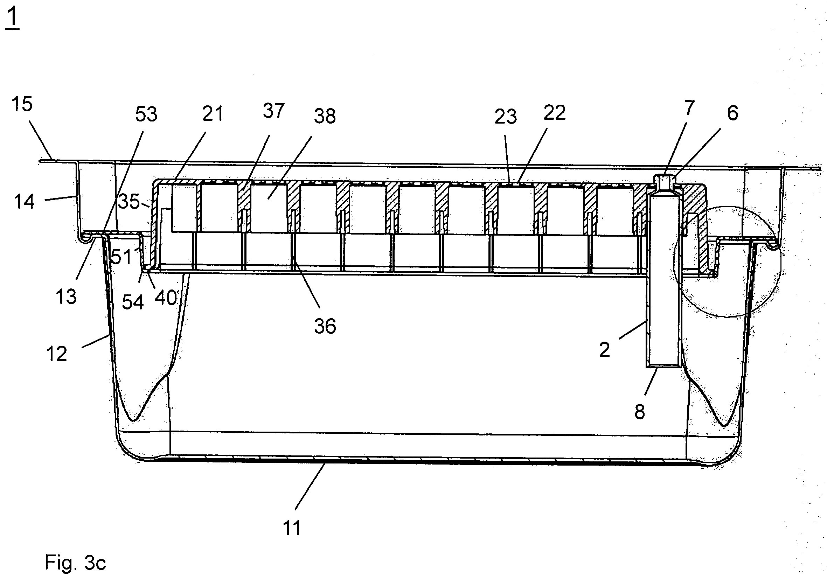

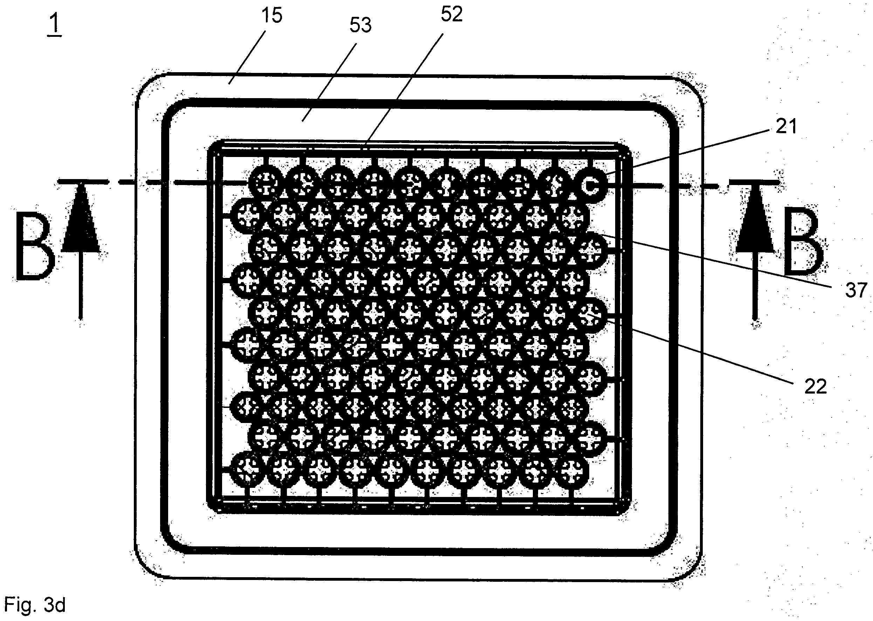

Die Fig. 2c zeigt einen stark vergrößerten Ausschnitt aus der Fig. 2b. In die Öffnungen 22 ragen jeweils vier elastische Haltezungen 23 hinein, die entlang dem Rand der Öffnungen 22 unter gleichen Winkelabständen zueinander angeordnet sind und jeweils über einen Schlitz 24 voneinander getrennt sind. Die Haltezungen 23 sind insbesondere einstückig mit der Platte 21 des Trägers 20 ausgebildet, was sich durch übliche 1K- oder 2K-Spritzgussverfahren in einfacher Weise realisieren lässt. Beim Einführen eines Behälters werden die Haltezungen 23 zunächst elastisch weggeschwenkt, um sich nach einem elastischen Zurückschwenken an den verengten Nackenabschnitt 5 der Behälter 2 anzuschmiegen (vgl. Fig. 3c) oder um die Behälter in diesem Bereich abzustützen, wie nachfolgend beschrieben. Weil die elastischen Haltezungen 23 in einer Anordnung mit mehrzähliger Punktsymmetrie in die Öffnungen 22 hineinragen, führt dies zu einer symmetrischen Kraftableitung beim Halten der Behälter über die Haltezungen 23, sodass die Behälter automatisch zentriert bezüglich einer Mittellinie einer jeweiligen Öffnung 22 gehalten werden.

Wie man der Fig. 2c entnehmen kann, ragen von der aufrecht und umlaufend ausgebildeten Seitenwand 35 des Trägers 20 eine Mehrzahl von Vorsprüngen 40 seitlich ab, die in einer vorbestimmten Geometrie durch Schlitze 41 unterbrochen sind, was jedoch nicht zwingend erforderlich ist.

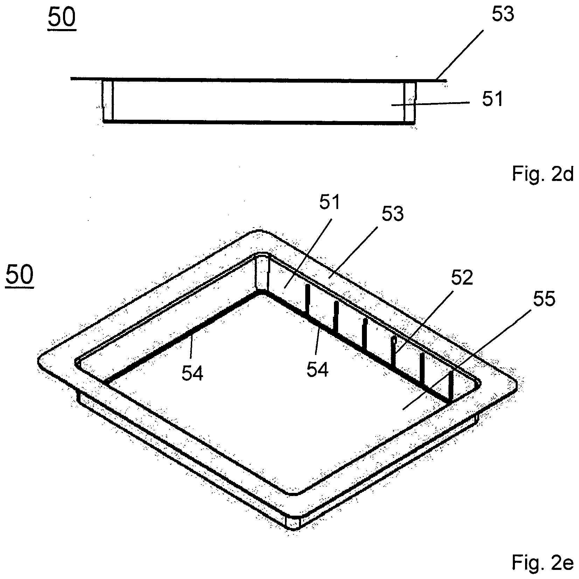

Die Figuren 2d und 2e zeigen einen Halterahmen zum Halten eines solchen Trägers 20, um eine Haltestruktur gemäß einer ersten Ausführungsform der vorliegenden Erfindung auszubilden. Gemäß der Fig. 2e ist der Rahmen 50 von einer umlaufenden Seitenwand 51 ausgebildet, die in einen flanschartigen, umlaufend ausgebildeten oberen Rand 53 übergeht. Der Rahmen ist auf der Ober- und Unterseite geöffnet ausgebildet, sodass die Seitenwand 51 eine Öffnung 55 umgibt. Gemäß der Fig. 2e sind auf der Innenseite der Seitenwand 51 eine Mehrzahl von Rippen 52 ausgebildet, die einerseits einer Versteifung des Rahmens 50 dienen können, andererseits auch aufgrund ihrer geometrischen Anordnung einer eindeutigen Festlegung der Lage eines Trägers 20, wie vorstehend beschrieben, in einem solchen Rahmen 50 dienen können.

Wie in der Fig. 2e dargestellt, brauchen sich die Rippen 52 nicht über die gesamte Höhe der Seitenwand 51 zu erstrecken, sodass diese gemeinsam eine Mehrzahl von Haltepunkten zur Abstützung des Trägers ausbilden. Gemäß der Fig. 2e ist am unteren Rand der Seitenwand 51 ein Vorsprung 54 ausgebildet, der in diesem Beispiel umlaufend ausgebildet ist (was nicht zwingend erforderlich ist) und einwärts in die Öffnung 55 hineinragt.

Der Rahmen 50 und der Träger 20 sind erfindungsgemäß so aufeinander abgestimmt, dass der Träger 20 in dem Rahmen 50 aufgenommen werden kann und die Vorsprünge 40 an der Seitenwand 35 des Trägers (vgl. Fig. 2c) in einer ersten Orientierung des Trägers unmittelbar auf den von den oberen Enden der Rippen 52 ausgebildeten Haltepunkten aufliegen. Wenn dagegen der Träger 20 gewendet wird und in einer zweiten Orientierung (d.h. mit der entgegengesetzten Orientierung) in den Rahmen 50 eingeführt wird, so greifen die Rippen 52 auf der Seitenwand 51 des Rahmens 50 in die Schlitze 41 zwischen den Vorsprüngen des Trägers 20 ein, sodass der Träger 20 entlang der Seitenwand 51 hinab gleiten kann, bis schließlich die Vorsprünge 40 auf dem unteren Rand 54 des Rahmens 20 aufliegen.

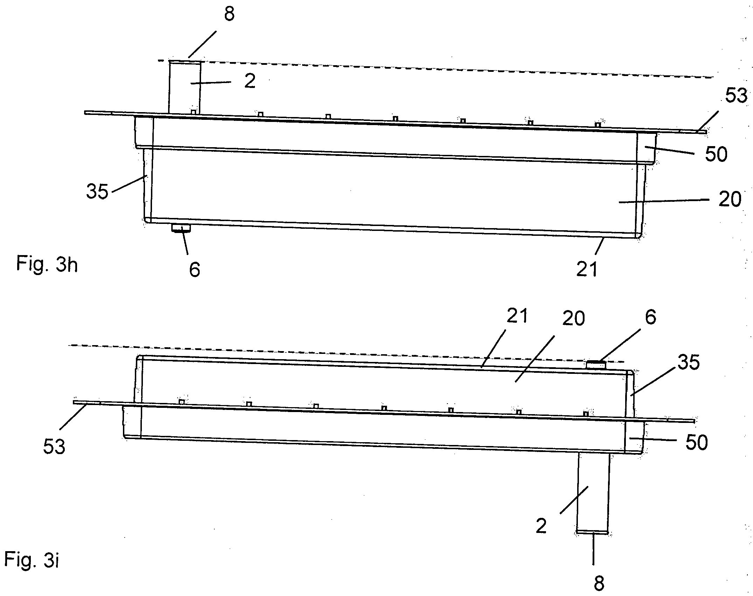

Die Haltezungen 23 sind erfindungsgemäß ausgelegt, um die Behälter in beiden Orientierungen des Trägers 20 (d.h. in beiden Stellungen der Behälter, also aufrecht oder kopfüber) axial an dem Träger 20 gesichert zu halten. Dies ist in einer Gegenüberstellung beispielhaft in den Figuren 3h und 3i dargestellt. Gemäß der Fig. 3h sind die Behälter kopfüber, d.h. mit ihrem verbreiterten oberen Rand 6 nach unten zeigend, so in die Öffnungen der Platte 21 der Trägers 20 eingesteckt, dass der obere Rand über die Platte 21 hinausragt und die Behälter im Bereich ihres verengten Nackenabschnitts an den Haltezungen der Platte 21 gehalten sind, wie vorstehend anhand der Fig. 2c beschrieben. Der Träger 20 ist von oben her so in den Halterahmen 53 eingeschoben, dass die Vorsprünge 40 an der Seitenwand des Trägers 20 (vgl. Fig. 2c) auf dem Vorsprung 54 am unteren Rand der Seitenwand 51 des Rahmens 50 (vgl. Fig. 2e) aufliegen. Wie durch die gestrichelte Linie in der Fig. 3h angedeutet, sind in dieser Stellung die unteren Enden der Behälter 2 (d.h. in der Fig. 3h die Befüllöffnungen der Karpulen 2) unter einem vorbestimmten Abstand zur Oberseite des oberen Rands 53 des Halterahmens 50 angeordnet.

In der entgegengesetzten Orientierung gemäß der Fig. 3i, wenn also der Träger 20 gewendet ist, was der ersten Stellung im Sinne des Anspruchs 1 entspricht, ist der Träger 20 von oben her so in den Halterahmen 53 eingeschoben, dass die Vorsprünge 40 an der Seitenwand des Trägers 20 (vgl. Fig. 2c) auf dem Vorsprung 54 am unteren Rand der Seitenwand 51 des Rahmens 50 (vgl. Fig. 2e) aufliegen. In dieser Orientierung sind die Karpulen 2 aufrecht in die Platte 21 des Trägers 20 eingehängt. D.h. die Karpulen 2 sind mit ihrer weiteren Öffnung am oberen Rand 6, die der Befüllung und der späteren Einführung eines Stopfens dient, nach oben zeigend und mit ihrer Auslassöffnung 8 nach unten zeigend in die Platte 21 des Trägers 20 eingehängt. Wie durch die gestrichelte Linie in der Fig. 3i angedeutet, sind in dieser Stellung der Behälter 2 die oberen Ränder der Behälter 2 (in der Fig. 3i die weiteren Öffnungen am oberen Rand 6 der Karpulen 2) unter dem gleichen vorbestimmten Abstand zur Oberseite des oberen Rands 53 des Halterahmens 50 angeordnet.

Entsprechendes gilt für den Abstand der oberen Enden 6 der Karpulen 2 zur Oberseite des oberen Rands 53 des Halterahmens 50 gemäß der Fig. 3h und für den Abstand der unteren Enden der Karpulen 2 zur Oberseite des oberen Rands 53 des Halterahmens 50 gemäß der Fig. 3i.

Somit ist die gemeinsam von dem Träger 20 und von dem Rahmen 50 ausgebildete Haltestruktur so auf die Länge der Behälter 2 abgestimmt, dass die oberen Enden 6 der Behälter 2 in der Stellung gemäß der Fig. 3i unter dem gleichen Abstand zur Oberseite des oberen Rands 53 des Halterahmens 50 angeordnet sind wie die unteren Enden 8 der Behälter 2 in der Stellung gemäß der Fig. 3h.

Wie man den Figuren 3h und 3i entnehmen kann, sind in beiden Orientierungen beide Enden der Behälter 2 für eine Behandlung oder Verarbeitung der Behälter 2 frei zugänglich, da in beiden Stellungen die Enden der Behälter 2 über den oberen bzw. unteren Rand der von dem Träger 20 und dem Rahmen 50 ausgebildete Haltestruktur hinaus ragen. Die Fig. 3h zeigt dabei den Rahmen 50 mit dem darin abgestützten Träger 20 und dem von diesen gehaltenen Behältern 2 in der zweiten Stellung im Sinne des Anspruchs 1.

Zum Transport und zur Verpackung der vorstehend beschriebenen Haltestruktur mit den darin aufgenommenen Behältern dient ein Transport- und Verpackungsbehälter 10, wie dieser schematisch in der Fig. 9a für einen einteilig ausgebildeten Träger dargestellt ist, der als Haltestruktur wirkt, jedoch nicht gemäß der vorliegenden Erfindung zweiteilig ausgebildet ist. Gemäß der Fig. 9a ist der Transport- und Verpackungsbehälter 10 im Wesentlichen kasten- oder wannenförmig ausgebildet und weist einen Boden 11, eine senkrecht von diesem abragende, umlaufend ausgebildete Seitenwand 12, eine von dieser im Wesentlichen rechtwinklig abragende Stufe 13, eine umlaufend ausgebildete obere Seitenwand 14 und einen oberen Rand 15 auf, der flanschartig ausgebildet ist Die Ecken 16 des Transport- und Verpackungsbehälters 10 sind zweckmäßig abgerundet ausgebildet. Die obere Seitenwand 14 kann unter einem geringen Neigungswinkel zur Senkrechten auf den Boden 11 geneigt ausgebildet sein, um das Einführen der von einem flächigen Träger 20 ausgebildeten Haltestruktur zu erleichtern. Ein derartiger Transport- und Verpackungsbehälter 10 ist bevorzugt aus einem Kunststoff ausgebildet, insbesondere durch Kunststoff-Spritzgusstechnik, und ist bevorzugt aus einem klaren, durchsichtigen Kunststoff ausgebildet, um eine optische Sichtkontrolle des Trägers 20, der in dem Transport- und Verpackungsbehälter 10 aufgenommenen ist, und der von dem Träger 20 gehaltenen Behälter 2 zu ermöglichen.

Zur zuverlässigen Positionierung des Trägers 20 in dem Transport- und Verpackungsbehälter 10 weisen der Träger und der Transport- und Verpackungsbehälter 10 miteinander zusammenwirkende Positionierungsgebilde auf, die insbesondere formschlüssig zusammenwirken. So können an geeigneter Stelle, insbesondere auf der Stufe 13 oder auf Abstützflächen 18 des Transport- und Verpackungsbehälters 10, Positionierungsgebilde in Form von Vorsprüngen oder Aussparungen bzw. Vertiefungen ausgebildet sein, die mit korrespondierend ausgebildeten Aussparungen bzw. Vertiefungen oder Vorsprüngen des Trägers 20 formschlüssig zusammenwirken, um den Träger 20 präzise in dem Transport- und Verpackungsbehälter 10 zu positionieren. Zu diesem Zweck können insbesondere auf der Stufe 13 des Transport- und Verpackungsbehälters 10 mehrere zapfenartige Vorsprünge (nicht gezeigt) ausgebildet sein, die in korrespondierend ausgebildete Zentrieröffnungen 27 in dem Träger 20 eingreifen.

Gemäß der Fig. 9a ist die Stufe 13 des Transport- und Verpackungsbehälters 10 als umlaufende, ebene Abstützfläche ausgebildet, auf welcher der Träger 20 unmittelbar aufliegt. Gemäß weiteren Ausführungsformen können an den Seitenwänden 12 des Transport- und Verpackungsbehälters 10 auch weitere Abstützflächen 18 oder Abstützelemente ausgebildet sein. Auf diese Weise kann der Träger 20 präzise in dem Transport- und Verpackungsbehälter 10 positioniert werden und die Mehrzahl von Fläschchen 2 auf diese Weise in einer regelmäßigen Anordnung und an präzise definierten Positionen in einem Transport- und Verpackungsbehälter 10 mit standardisierten Abmessungen angeordnet und gehalten werden. Insbesondere kann auf diese Weise gewährleistet werden, dass sämtliche Böden oder untere Enden der Fläschchen 2 in einer gemeinsam aufgespannten Ebene parallel zum Boden 11 oder zum oberen Rand 15 des Transport- und Verpackungsbehälters 10 angeordnet sind.

Wenngleich in der Fig. 9a der Boden 11 des Transport- und Verpackungsbehälters 10 als geschlossen und einstückig mit der Seitenwand 12 ausgebildet dargestellt ist, kann das untere Ende des Transport- und Verpackungsbehälters 10 auch in der Art des oberen Endes geöffnet ausgebildet sein, insbesondere mit einem flanschartigen unteren Rand in der Art des oberen Rands 15 versehen sein, so dass die Böden der Fläschchen 2 von der Unterseite des Transport- und Verpackungsbehälters 10 her frei zugänglich sind, beispielsweise für Verarbeitungsschritte in einem Steriltunnel oder in einem Gefriertrockenschrank, wie nachfolgend näher erläutert.

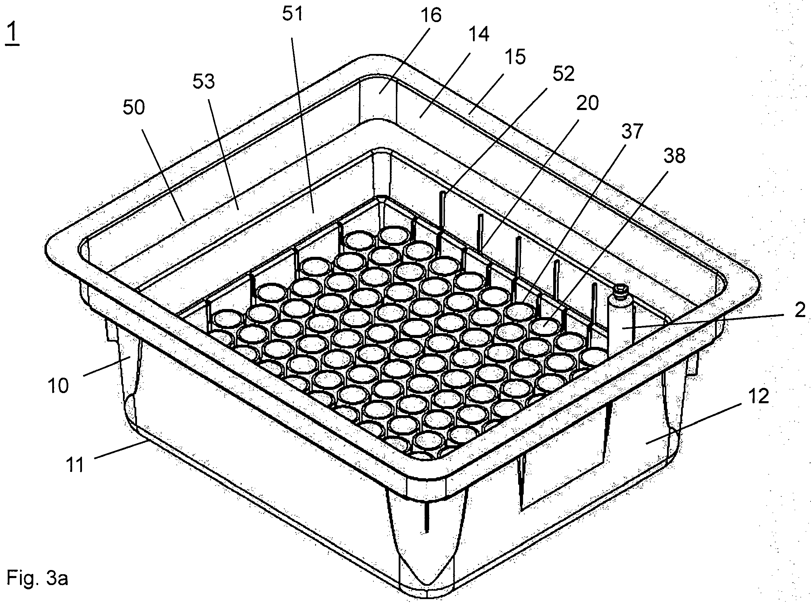

Die Aufnahme einer Haltestruktur, die zweiteilig ist und von einem Halterahmen 50 und einem Träger 20 gemäß den Figuren 2a bis 2g ausgebildet ist, in einem Transport- und Verpackungsbehälter 10, wie vorstehend beschrieben, ist in den Figuren 3a bis 3g dargestellt. Die Haltestruktur kann dabei unmittelbar auf dem Boden 11 des Transport- und Verpackungsbehälters 10 abgestützt sein oder kann auf Abstützflächen des Transport- und Verpackungsbehälters 10 abgestützt sein. Beispielsweise kann der obere Rand 53 des Halterahmens 50 auf den oberen Enden von Vorsprüngen abgestützt sein, die auf der Seitenwand 12 oder auf dem Boden 11 des Transport- und Verpackungsbehälters 10 vorgesehen sind. Auf diese Weise lassen sich erfindungsgemäß auch mehr als zwei Höhenniveaus der Enden der an dem Träger 20 gehaltenen Behälter 2 realisieren.

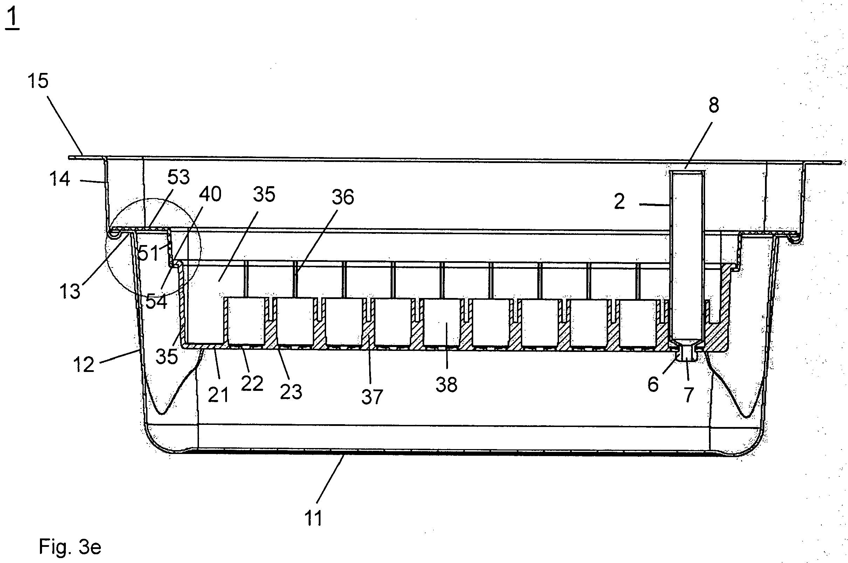

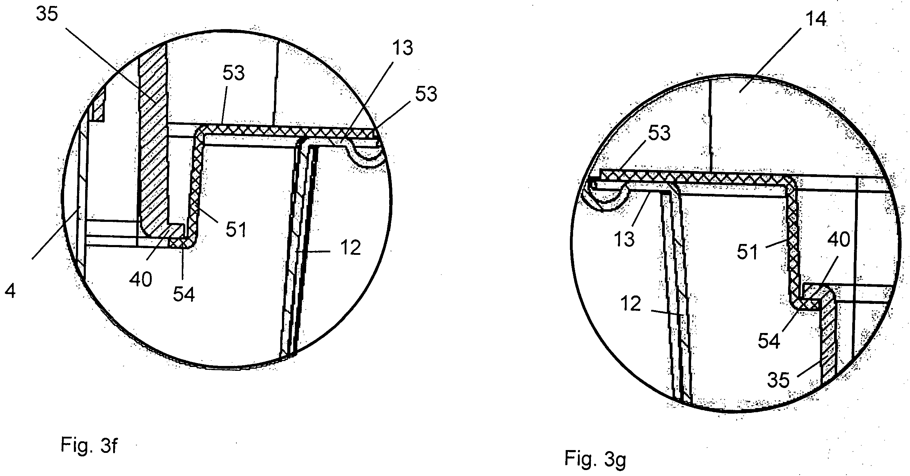

Die Fig. 3f zeigt dabei den in der Fig. 3c umkreisten Teilausschnitt in einer stark vergrößerten Darstellung, und zwar in einer Stellung, in der der Träger 20 so in den Rahmen 50 eingeführt ist, dass die Karpulen 2 aufrecht in die Öffnungen 22 der Platte 21 des Trägers 20 eingehängt sind. Die Fig. 3g zeigt hingegen den in der Fig. 3e eingekreisten Teilausschnitt in einer stark vergrößerten Darstellung, und zwar in einer Stellung, in der der Träger 20 so in den Rahmen 50 eingeführt ist, dass die Karpulen 2 kopfüber in die Öffnungen 22 der Platte 21 des Trägers 20 eingesteckt sind und die Karpulen 2 auf der Oberseite des Trägers 20 angeordnet sind.

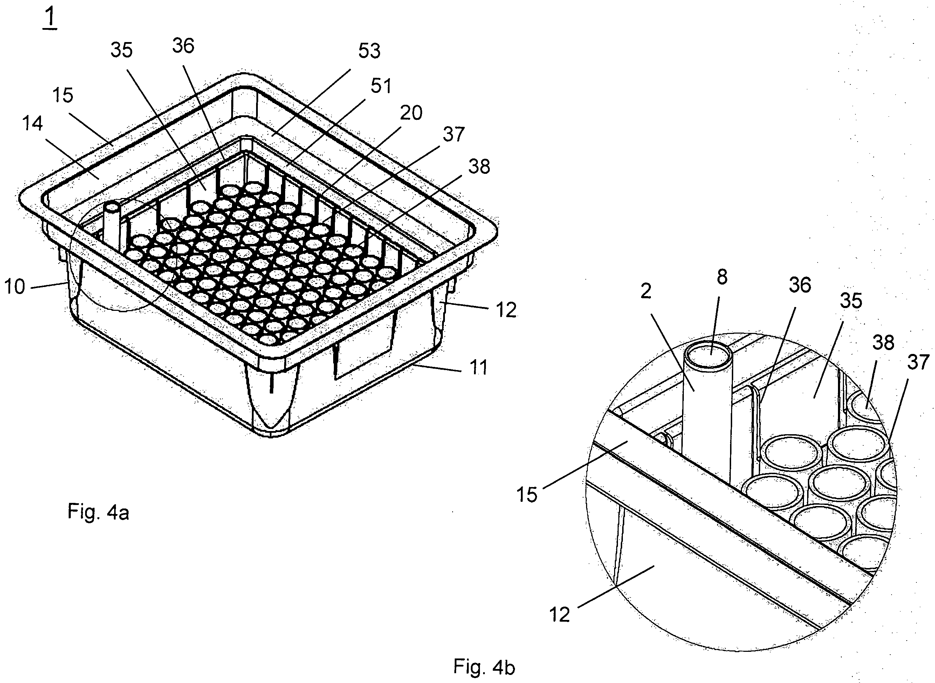

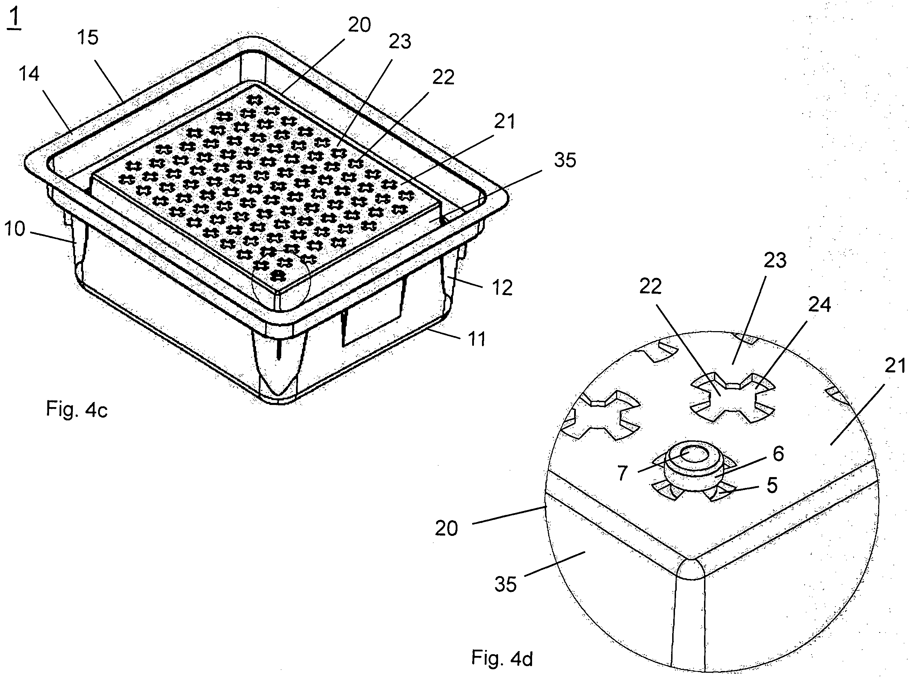

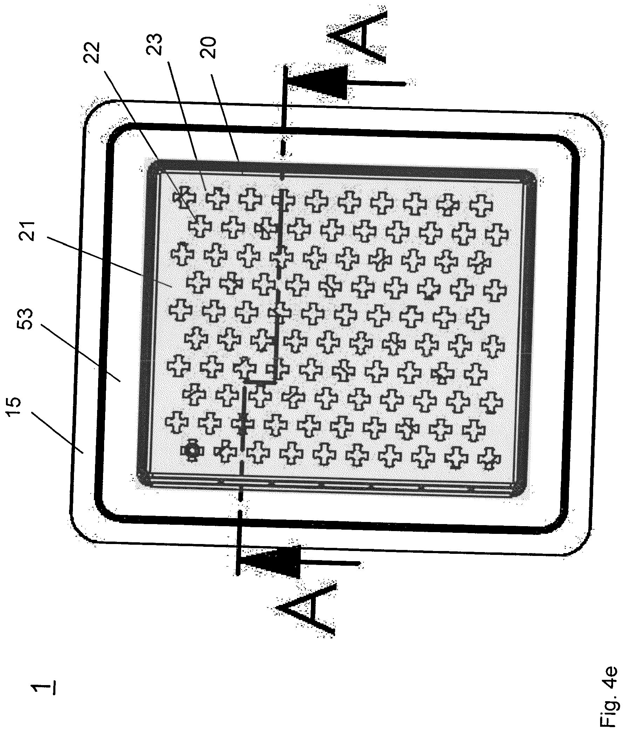

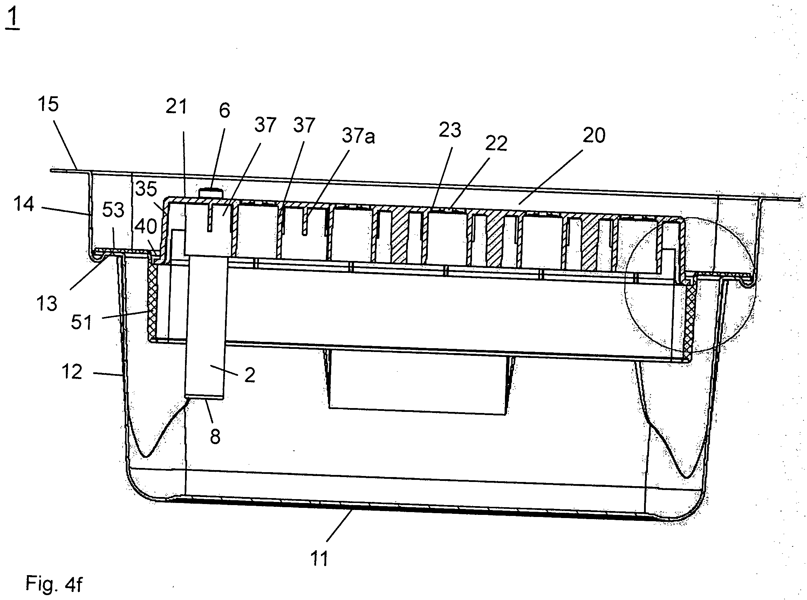

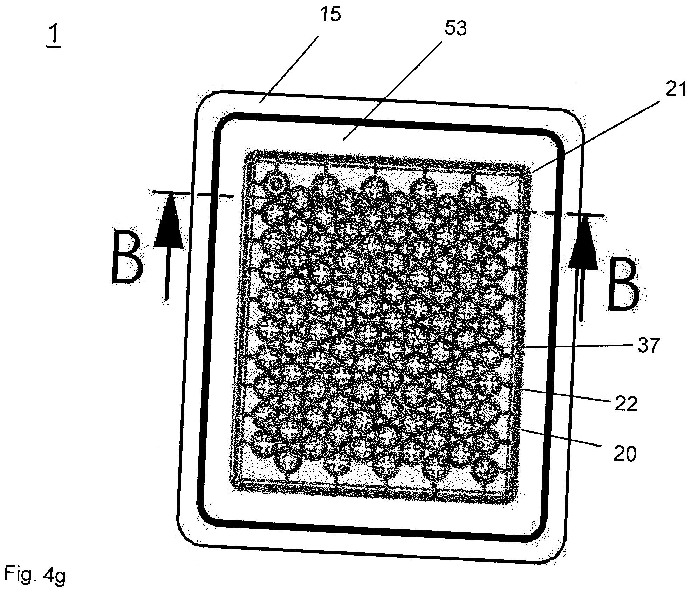

Die Figuren 4a bis 4j zeigen die Aufnahme einer Haltestruktur gemäß einer weiteren Ausführungsform in einem Transport- und Verpackungsbehälter 10, wie vorstehend beschrieben und mit Anspruch 2 beansprucht. Wie in den Figuren 4c, 4d und 4f gezeigt, können die vorderen Enden der als Karpulen 2 ausgebildeten Behälter so in die Öffnungen der Platte 21 des Trägers 20 eingesteckt werden, dass der verbreiterte obere Rand etwas über die Platte 21 hinausragt und dass die Haltezungen die Karpulen 2 jeweils im Bereich des verengten Nackenabschnitts abstützen oder halten. Dabei erstrecken sich die vorderen Enden der Karpulen durch die von den zylindrischen Seitenwänden 37 ausgebildeten Aufnahmen. Die Seitenwände 37 sind dabei mit einem Schlitz 37a versehen und können somit geringfügig aufgeweitet werden, etwa um die vorderen Enden der Karpulen zu führen oder zu klemmen. Die Seitenwände 37 verhindern dabei eine Kollision von unmittelbar benachbart zueinander an dem Träger 20 gehaltenen Karpulen 2.

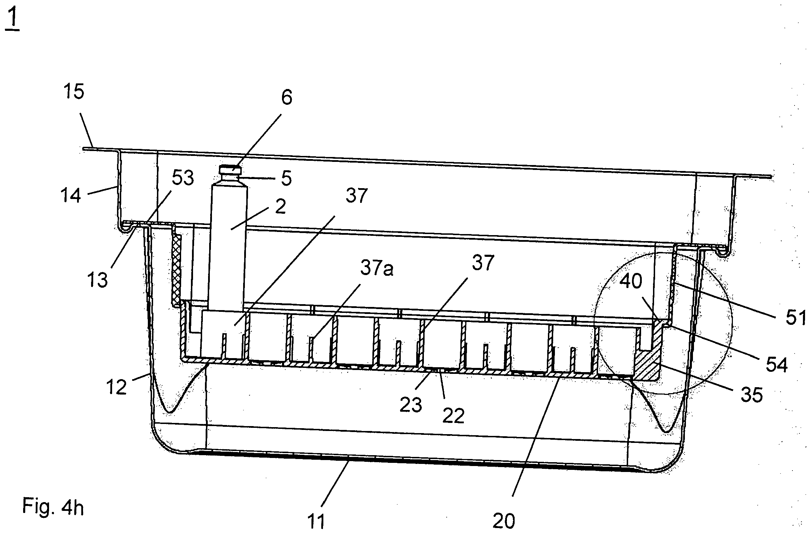

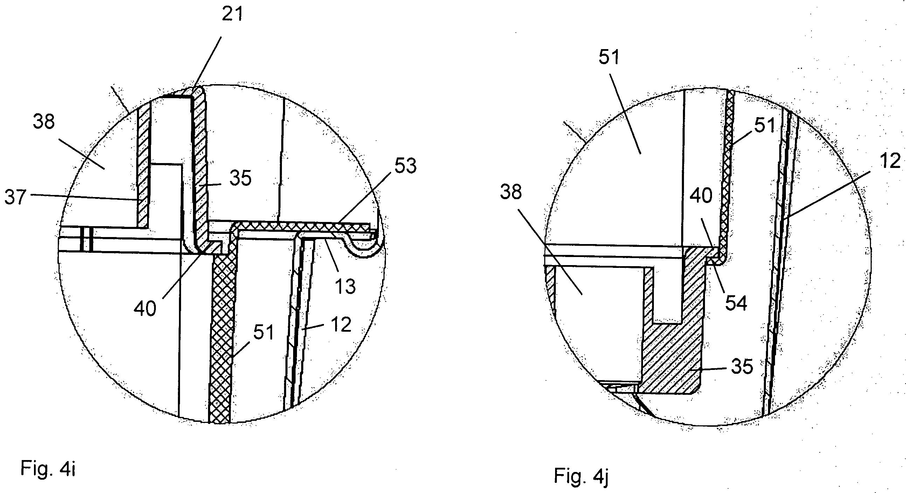

Die Fig. 4i zeigt dabei den in der Fig. 4f eingekreisten Teilausschnitt in einer stark vergrößerten Darstellung, und zwar in einer Stellung, in der der Träger 20 so in den Rahmen 50 eingeführt ist, dass die Karpulen 2 aufrecht in die Öffnungen 22 der Platte 21 des Trägers 20 eingehängt sind, was der zweiten Stellung im Sinne des Anspruchs 2 entspricht. In der Stellung gemäß der Fig. 4h sind die unteren Enden der Karpulen 2 dagegen in die von den Seitenwänden 37 ausgebildeten Aufnahmen eingesteckt, sodass diese unmittelbar auf der Platte 21 des Trägers 20 aufliegen, was der ersten Stellung im Sinne des Anspruchs 2 entspricht. Die Seitenwände 37 können dabei die unteren Enden der Karpulen 2 auch geringfügig klemmen.

Die Fig. 4j zeigt den in der Fig. 4h eingekreisten Teilausschnitt in einer stark vergrößerten Darstellung, und zwar in einer Stellung, in der der Träger 20 so in den Rahmen 50 eingeführt ist, dass die unteren Enden der Karpulen 2 in die von den Seitenwänden 37 ausgebildeten Aufnahmen eingesteckt sind und die oberen Enden der Karpulen 2 mit den dort vorgesehenen verbreiterten Rändern 6 ebenfalls zu der oberen Einführöffnung des Transport- und Verpackungsbehälters 10 hin gerichtet sind, was der ersten Stellung im Sinne des Anspruchs 2 entspricht. Weil die vorderen Enden der Karpulen 2 in dieser Stellung nicht in die Öffnungen der Platte 21 des Trägers 20 eingesteckt sind, ist der Abstand des oberen Rands 6 der Karpulen 2 in der Stellung gemäß der Fig. 4h relativ zu der Platte 21 des Trägers 20 geringfügig größer als der Abstand des unteren Rands 8 der Karpulen 2 zu der Platte 21 des Trägers 20 in der Stellung gemäß der Fig. 4f. Diese Höhendifferenz entspricht im Wesentlichen der axialen Länge des verengten Halsabschnitts am oberen Rand der Karpulen 2 und ist somit im Vergleich zur Gesamtlänge der Karpulen 2 im Sinne der vorliegenden Anmeldung vernachlässigbar.

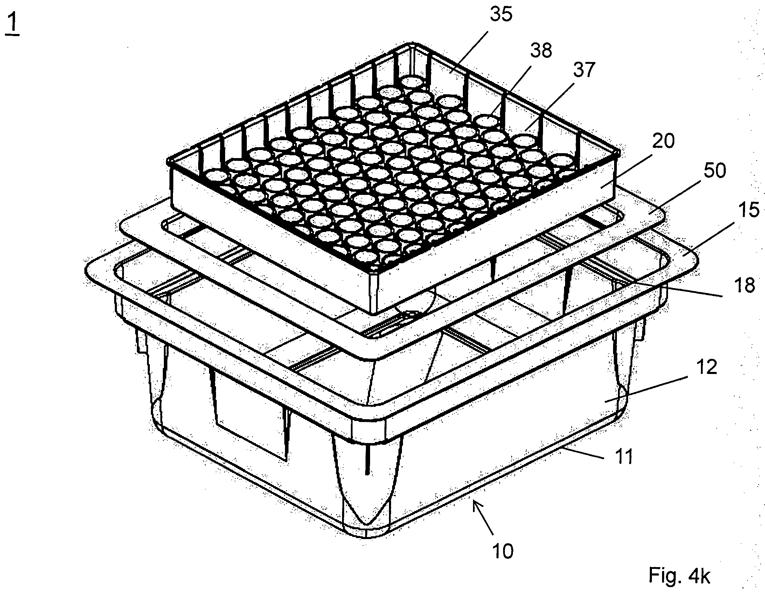

Die Fig. 4k zeigt den vorstehend beschriebenen Transport- und Verpackungsbehälter in einer perspektivischen Explosionsansicht.

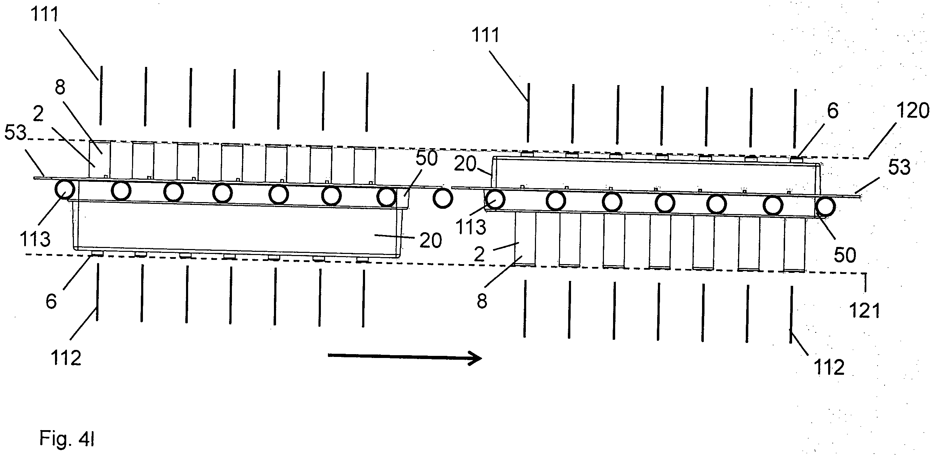

Die Fig. 4l zeigt in einer schematischen Darstellung ein Verfahren zur gleichzeitigen Behandlung oder Verarbeitung einer Mehrzahl von Behältern gemäß der vorliegenden Erfindung. Im linken und rechten Bildteil ist dabei jeweils eine Haltestruktur dargestellt, wobei die Behälter 2 im linken Bildteil mit entgegen gesetzter Orientierung an dem Träger 20 gehalten sind als im rechten Bildteil. Die Haltestrukturen werden mittels einer Fördereinrichtung 113, beispielsweise mit Hilfe von Förderwalzen oder Förderketten, in die Richtung des Pfeils gefördert, also in der Fig. 4l von links nach rechts. Die Fördereinrichtung 113 kann beispielsweise auf den oberen Rand 53 des Halterahmens 50 einwirken. Aus Vereinfachungsgründen ist in der Fig. 4l der zugeordnete Transport- und Verpackungsbehälter nicht dargestellt. Dem Fachmann wird jedoch beim Studium der vorstehenden Beschreibung ohne Weiteres ersichtlich sein, dass die Behandlung oder Verarbeitung der Behälter wahlweise ohne einen solchen Transport- und Verpackungsbehälter erfolgen kann oder während die Haltestruktur in dem Transport- und Verpackungsbehälter aufgenommen ist. Sofern der Boden des Transport- und Verpackungsbehälters dann geschlossen ausgebildet ist, wie beispielsweise in der Fig. 9a abgebildet, kann die Behandlung oder Verarbeitung der Behälter dann nur von der oberen Einführöffnung des Transport- und Verpackungsbehälters her erfolgen.

Gemäß der Fig. 4l werden die an der Haltestruktur gehaltenen Behälter 2 zur Behandlung oder Verarbeitung automatisiert an Prozessstationen 111, 112 vorbeigeführt, die oberhalb und/oder unterhalb der Fördereinrichtung 113 angeordnet sein können. Wie man der Fig. 4l entnehmen kann, brauchen die Prozessstationen 111, 112 unabhängig davon, ob die Behälter 2 an der Haltestruktur in der ersten Stellung (also beispielsweise aufrecht stehend) oder in der zweiten Stellung (also beispielsweise kopfüber) gehalten sind, in ihrer Höhe nicht verstellt werden, damit die Behälter 2 behandelt oder zu verarbeitet werden können, weil sich die oberen und unteren Enden in beiden Stellungen (Orientierungen), abgesehen von den unvermeidlichen Toleranzen, wie vorstehend ausgeführt, auf dem gleichen Höhenniveau befinden. Dies erleichtert erfindungsgemäß die Behandlung und Verarbeitung der Behälter erheblich, weil der Aufwand hinsichtlich Justierung, Steuerung und Automatisierung der Prozessstationen 111, 112 und der Fördereinrichtung 113 erheblich vereinfacht werden kann.

Die Fig. 5a zeigt eine weitere Ausführungsform eines Trägers für eine Haltestruktur gemäß der vorliegenden Erfindung, bei dem die Haltezungen 23 als elastische Haltezungen ausgebildet sind, die von der Oberseite der Platte 21 des Trägers bogenförmig abragen und radial einwärts in die zugeordneten Öffnungen der Platte 21 des Trägers hineinragen. Erkennbar ist insbesondere, dass in der Ruhelage bzw. Haltestellung die Karpulen bevorzugt so gehalten sind, dass ein radiales Spiel zwischen den vorderen Enden der Haltezungen 23 und dem verengten Nackenabschnitt 5 der Karpulen vorhanden ist und dass somit in der Stellung gemäß dem linken Bildteil der Fig. 5a die Unterseite des verbreiterten oberen Rands 6 der Karpulen auf den vorderen Enden der Haltezungen 23 aufliegen oder dass in der Stellung gemäß dem rechten Bildteil der Fig. 5a der Übergangsbereich zwischen dem verengten Nackenabschnitt 5 und der zylindrischen Seitenwand 4 der Karpulen auf der Rückseite der elastischen Haltezungen 23 lose aufliegen. Auf diese Weise können die Karpulen in beiden Orientierungen in einer Richtung axial gesichert an der Platte 21 gehalten werden. Die gestrichelte Linie deutet dabei in der Fig. 5a das gleiche Höhenniveau des oberen Rands 6 der Karpulen und des unteren Endes 8 der Karpulen an, wenn diese an der Platte 21 gehalten sind.

Die Fig. 5b zeigt in einem Teilschnitt die Haltezunge eines Trägers einer Haltestruktur gemäß einer weiteren Ausführungsform. Erkennbar sind die elastischen Haltezungen 23 fähnchenartig und mit einer radial einwärts vorstehenden Haltenase ausgebildet. Gemäß der Fig. 5b sind die elastischen Haltezungen 23 über eine senkrecht von der Oberseite der Platte 21 des Trägers abragende, elastische Basis 23a mit der Platte 21 verbunden. Die Basis 23a geht über in einen radial einwärts gekrümmten Abschnitt 23b über, der schließlich in die Haltenase 23 c übergeht, auf welcher der verbreiterte Rand 6 (vgl. Fig. 1) der Behälter aufliegt. Die Haltenase 23c ragt dabei in die Öffnung der Platte 21 des Trägers hinein. Die Haltenase 23c geht in eine sich schräg aufwärts erstreckende Einführschräge 23f über, die mit dem oberen Ende der Haltezunge 23 verbindet. Zwischen der oberen Einführschräge 23f und der unteren Einführschräge 23b ist eine Aussparung 23d ausgebildet, in der der obere Rand 6 in beiden Richtungen axial gesichert (also geeignet für eine aufrechte Orientierung oder eine Orientierung kopfüber) und mit radialem Spiel aufgenommen ist. Aufgrund der Einführschräge 23f auf der Oberseite der Haltezunge 23 sowie des nach unten geöffneten, gekrümmten Abschnitts 23b der Haltezunge 23 können die Behälter wahlweise von oben oder von unten her in die Öffnungen der Platte 21 des Trägers eingeführt und aus diesen wieder abgezogen werden.

Beim Einführen der Behälter von oben her in die Öffnungen des Trägers geraten zunächst die Böden bzw. unteren Enden der Behälter in Anlage zu den Einführschrägen 23f der Haltezungen 23. Beim weiteren Einführen der Behälter gleitet das untere Ende bzw. der Boden der Behälter entlang den Einführschrägen 23f abwärts und spreizt dabei die Haltezungen 23 zunehmend elastisch auseinander oder klappt bzw. schwenkt diese zurück. Beim weiteren Einführen der Behälter gerät schließlich die zylindrische Seitenwand 4 der Behälter in Anlage zu den Haltenasen 23e und gleitet an diesen entlang, solange bis schließlich die Unterseite des verbreiterten Rands 6 der Behälter lose auf den Haltenasen 23c der Haltezungen 23 aufliegt. Die Behälter können dann entweder nach oben hin mit umgekehrtem Bewegungsablauf der Haltezungen 23 oder nach unten hin mit elastischem Verbiegen der Haltezungen 23 aus den Öffnungen der Platte 21 des Trägers entnommen werden.

Beim Einführen der Behälter von unten her in die Öffnungen des Trägers gerät das obere Ende der Behälter zunächst in Anlage zu dem gekrümmten Abschnitt 23b der Haltezungen 23. Beim weiteren Einführen der Behälter gleitet das obere Ende der Behälter entlang den gekrümmten Abschnitten 23b aufwärts und spreizt dabei die Haltezungen 23 zunehmend elastisch auseinander oder klappt bzw. schwenkt diese zurück, bis schließlich die Haltenasen 23c erreicht sind. Beim weiteren Hochschieben der Behälter gleitet die Unterseite des verbreiterten Rands 6 der Behälter über die Haltenasen 23c der Haltezungen 23 und liegt schließlich lose auf den Haltenasen 23c der Haltezungen 23 auf. Die Behälter können dann entweder nach unten hin mit umgekehrtem Bewegungsablauf der Haltezungen 140 oder nach oben hin mit elastischem Verbiegen der Haltezungen 23 aus den Öffnungen der Platte 21 des Trägers entnommen werden.

Bei der Ausführungsform nach der Fig. 5b wird somit der verbreiterte obere Randabschnitt 6 (Rollrand) der Behälter klammerartig und formschlüssig umgriffen, wobei ein ausreichendes radiales Spiel, wie vorstehend beschrieben, gewährleistet ist, wie durch den Luftspalt in Radialrichtung in der Fig. 5b angedeutet. Ergänzend kann auch ein ausreichendes axiales Spiel gewährleistet sein, wie durch den Luftspalt in axialer Richtung in der Fig. 5b angedeutet.

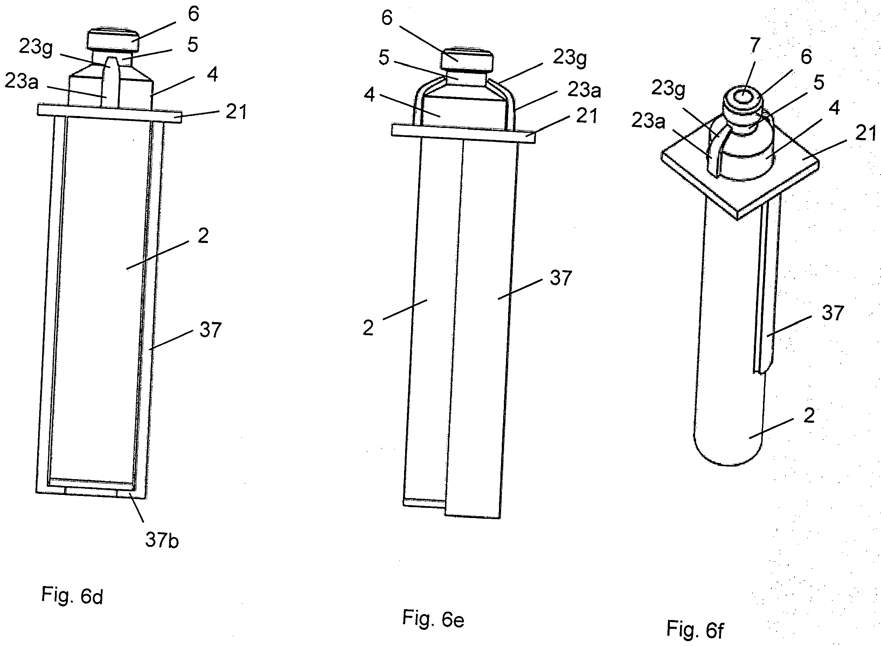

Die Figuren 6a bis 6h zeigen weitere Varianten zur Halterung der Behälter an einem Träger, die ergänzend oder alternativ zu den vorstehend beschriebenen Halterungen vorgesehen sein können. Die Fig. 6a zeigt eine als Halbschale ausgebildete Seitenwand 37, die von der Unterseite der Platte 21 des Trägers abragt und an ihrem unteren Ende einen radial einwärts abragenden Vorsprung 37b aufweist, in dem eine Aussparung ausgebildet ist. Dieser Vorsprung 37b kann als Anschlag zur Festlegung der axialen Position des Behälters 2 an dem Träger verwendet werden, wie in der Fig. 6b abgebildet, in der die Unterseite des verbreiterten oberen Rands 6 der Behälters 2 lose und bevorzugt mit radialem Spiel auf den vorderen Enden 23g der Haltezungen 23a aufliegt. Die Fig. 6c zeigt eine Seitenansicht der Halterung nach der Fig. 6b. Wie man in der Fig. 6c erkennt, ist aufgrund der Ausgestaltung der Seitenwand 37 als Halbschale die gesamte linke Hälfte des Behälters 2 von der Seite der Haltestruktur her frei zugänglich, beispielsweise für eine Bearbeitung, Handhabung oder für eine (optische) Inspektion des Behälters oder seines Inhalts. Da den Öffnungen des Trägers jeweils nur zwei Haltezungen 23a (auf einander diametrial gegenüber liegenden Seiten der Öffnungen) zugeordnet sind, ist auch das gesamte obere Ende der Behälter 2, einschließlich des verengten Nackenabschnitts 5, von der Seite des Trägers her frei zugänglich, beispielsweise für eine Bearbeitung, Handhabung oder für eine (optische) Inspektion des Behälters oder seines Inhalts, wie nachfolgend anhand der Fig. 6i beschrieben. Die Figuren 6d und 6e zeigen weitere Seitenansichten dieser Haltestruktur.

Die Fig. 6f zeigt schließlich eine weitere Ausführungsform, bei der die Seitenwand 37 kürzer als die Seitenwand des Behälters 2 ausgebildet ist und somit das gesamte untere Ende der Behälter 2 von der Unterseite des Trägers her frei zugänglich ist. Im Falle der Ausführungsform nach der Fig. 6a ist bei Verwendung einer Karpule dagegen der untere Rand der Karpule auf dem Vorsprung 37b abgestützt, jedoch die Befüllöffnung am unteren Ende der Karpule frei zugänglich, beispielsweise für eine Befüllung der Karpule.

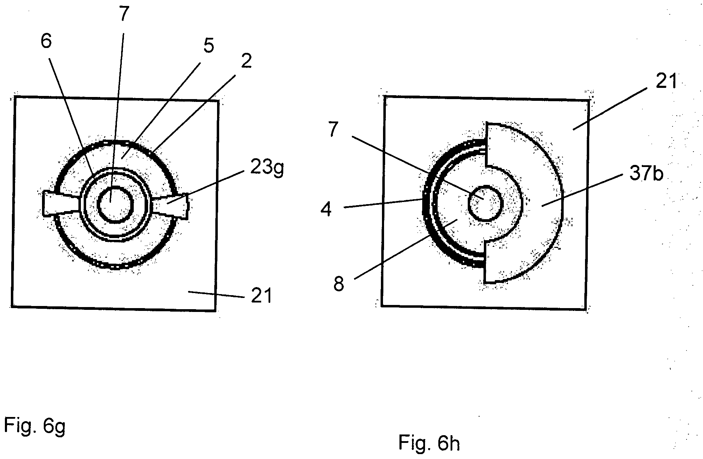

Die Figuren 6g und 6h zeigen diese Haltestruktur in einer Draufsicht und einer Unteransicht.

Die Fig. 6i zeigt beispielhaft die Messung des Füllstands bzw. der Füllmenge eines als Karpule ausgebildeten Behälters mit Hilfe eines Lasers 100. Es sei angenommen, dass die Karpule über die Einfüllöffnung am oberen Rand 6 mittels einer Injektionsnadel 110 befüllt wird. Der schraffierte Bereich 9a deutet den bereits gefüllten Teil der Karpule an, während oberhalb dieses Bereichs ein noch nicht gefüllter Bereich 9b, beispielsweise mit Luft oder einem Inertgas gefüllt, vorliegt. Der Laserstrahl 101 des Lasers 100 durchstrahlt den verengten Nackenabschnitt 5 der Karpule. Der Laserstrahl 101 wird auf der gegenüber liegenden Seite der Karpule mit Hilfe eines optischen Sensors 102 detektiert. Wenn die Karpule auch im Bereich des Laserstrahls 101 gefüllt ist, tritt aufgrund der anderen optischen Bedingungen entlang des Strahlwegs ein seitlicher Versatz oder eine Ablenkung des Laserstrahls 101 auf, der mit dem Sensor 102 detektiert werden kann. Da der Laserstrahl 101 auf einen relativ geringen Strahldurchmesser kollimiert werden kann, kann so auch die aktuelle Füllmenge der Karpule präzise detektiert werden und beispielsweise bei Erreichen eines vorbestimmten Füllstands eine Bearbeitung oder Verarbeitung der Karpule, beispielsweise ein Befüllvorgang, abgebrochen werden.

Die Figuren 7a bis 7f zeigen eine Haltestruktur, gemäß einer weiteren Ausführungsform zum besseren Verständnis der vorliegenden Erfindung. Statt der vorstehend beschriebenen Haltezungen ragen bei dieser Ausführungsform von der Platte des Trägers 20 zylindrische oder hohlzylindrische Zapfen 42 im Wesentlichen senkrecht ab, um eine Berührung von unmittelbar benachbart an dem Träger 20 gehaltenen Behältern zu verhindern.

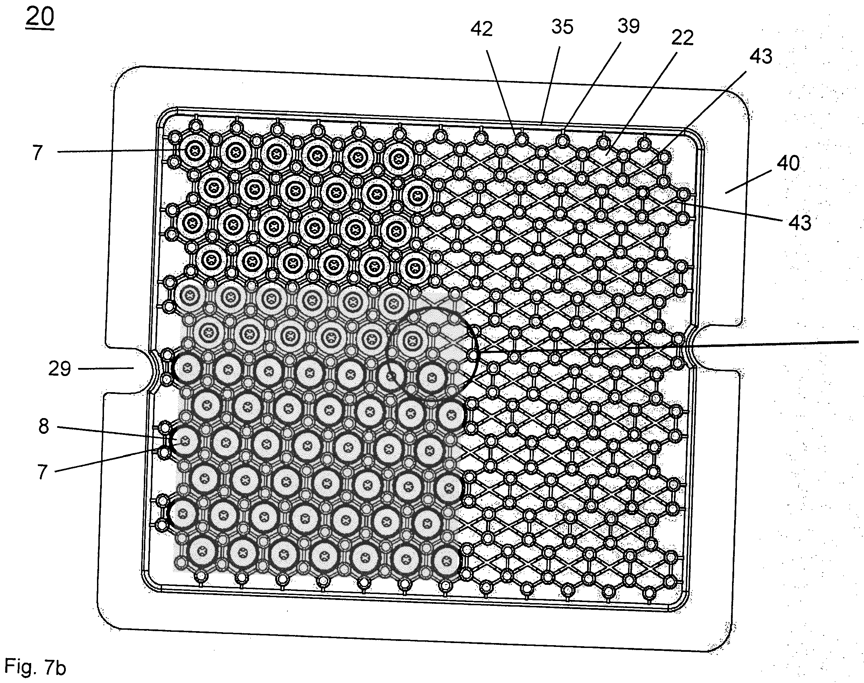

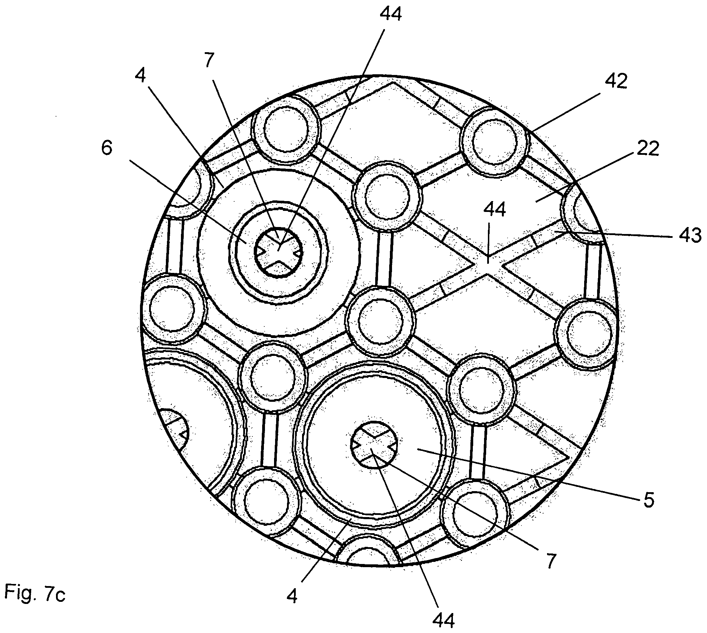

In der Draufsicht gemäß der Fig. 7b und in der stark vergrößerten Teildarstellung gemäß der Fig. 7c erkennt man, dass der Träger 20 im Wesentlichen offen ausgebildet ist und von sich kreuzenden Haltestegen 43 überspannt ist, die einen netzartigen Boden des Trägers ausbilden, auf welchem die Zapfen 42 angeordnet sind. Gemäß der Fig. 7c bilden jeweils sechs Haltezapfen 42 eine Aufnahme aus, in der das untere oder obere Ende der Behälter aufgenommen ist. Dabei kann zwischen den Haltezapfen 42 und der Seitenwand 4 der Behälter ein radiales Spiel vorhanden sein. Oder die Haltezapfen 42 können an der Seitenwand 4 der Behälter tangential anliegen und diese ggf. sogar geringfügig klemmen. Die sich kreuzenden Haltestege 43 stützen dabei die jeweiligen Enden der Behälter ab.

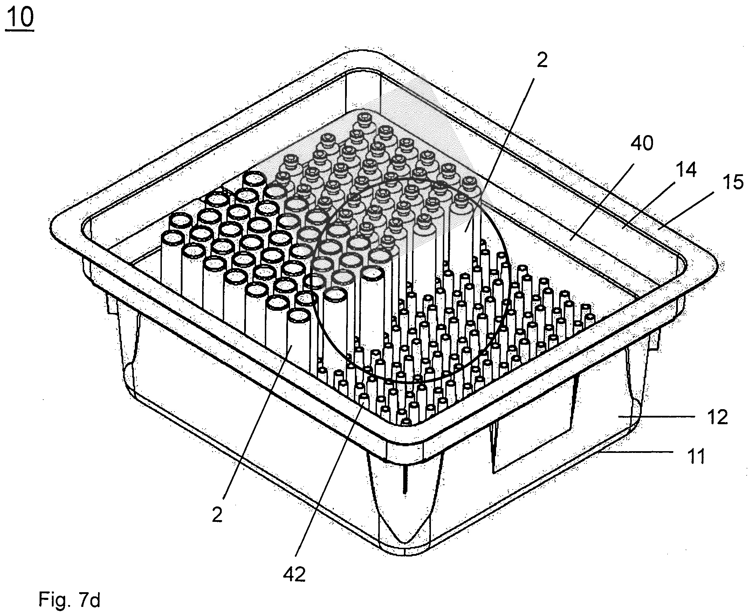

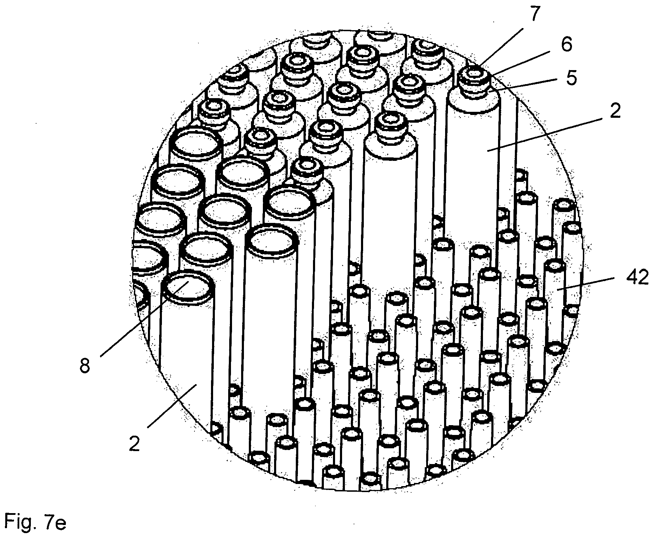

Wie man der Fig. 7d und der stark vergrößerten Teildarstellung gemäß der Fig. 7e entnehmen kann, können die Behälter 2 (in den Figuren als Karpulen dargestellt) in die Aufnahmen, die von den Zapfen 42 ausgebildet werden, aufrecht oder kopfüber eingesteckt sein. Gemäß der Fig. 7d ist ein so ausgebildeter Träger unmittelbar in dem Transport- und Verpackungsbehälter 10 aufgenommen.

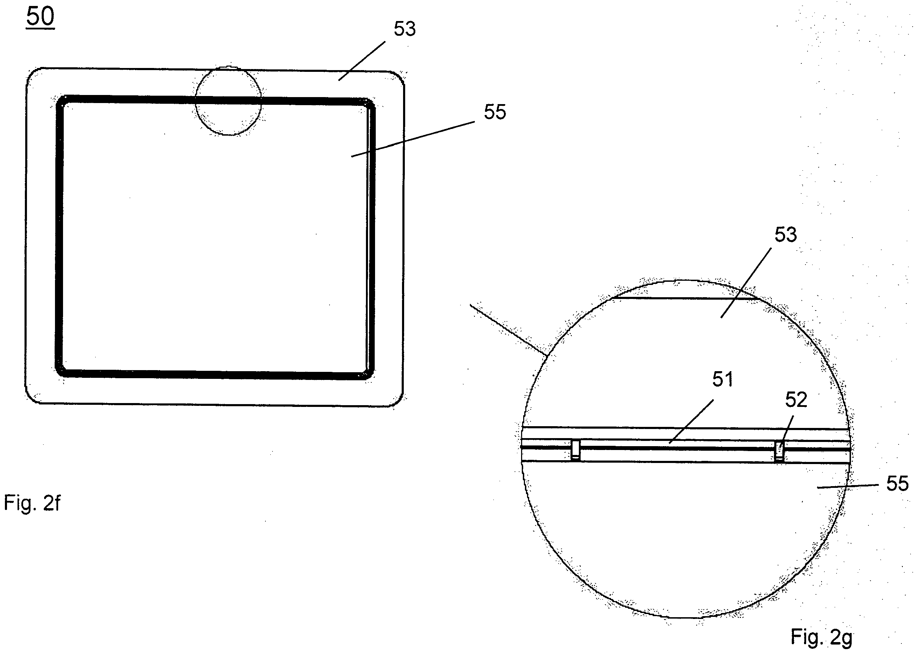

Bei einer Ausführungsform nach der vorliegenden Erfindung ist bei einer solchen Haltestruktur zusätzlich auch ein zusätzlicher Halterahmen (nicht gezeigt) vorgesehen, in welchem der Träger seinerseits aufgenommen ist, wie vorstehend anhand der Figuren 2 beschrieben, um eine zweiteilige Haltestruktur, auszubilden. Die Fig. 7f zeigt einen Transport-und Verpackungsbehälter 10 gemäß einer solchen weiteren Ausführungsform in einer perspektivischen Explosionsdarstellung.

Gemäß der Fig. 7c kann im Bereich eines Kreuzungspunkts 44 der sich kreuzenden Haltestege 43 ein vertiefter Abschnitt ausgebildet sein, um eine Aufnahme für einen verengten Halsabschnitt 5 der zu haltenden Behälter (vgl. Fig. 1) auszubilden, um einem Verkippen der an dem Träger gehaltenen Behälter entgegen zu wirken. Dadurch kommt es zu einer geringfügigen Differenz zwischen dem Abstand, unter dem die oberen Enden 6 der Behälter 2 in der ersten Stellung zu dem Träger 2 angeordnet sind, und dem Abstand, unter dem die unteren Enden 3, 8 der Behälter 2 in der zweiten Stellung zu dem Träger 20 angeordnet sind. Diese entspricht jedoch im Wesentlichen der axialen Länge des verengten Halsabschnitts 5 der zu haltenden Behälter und ist somit im Vergleich zur Gesamtlänge der Behälter üblicherweise vernachlässigbar.

Die Fig. 8a zeigt in einer Draufsicht eine Haltestruktur gemäß einer weiteren Ausführungsform der vorliegenden Erfindung. Wie auch bei der Ausführungsform nach der Fig. 7a ragen gemäß der Fig. 8a von dem Boden 21 des Trägers 20 zylindrische oder hohlzylindrische Zapfen 42 im Wesentlichen senkrecht ab, um eine Berührung von unmittelbar benachbarten Behältern, die an dem Träger 20 gehalten werden, zu verhindern. Die Zapfen 42 sind über Stege 47 miteinander verbunden, die einer Versteifung des Bodens 21 dienen sollen. Insgesamt sind um die jeweiligen Öffnungen 22 des Trägers 20 herum jeweils sechs Zapfen 42 unter gleichen Winkelabständen zueinander angeordnet, um eine Aufnahme auszubilden, in der ein Behälter aufgenommen werden soll. Der Boden 21 des Trägers 20 ist, abgesehen von den Öffnungen 22, geschlossen ausgebildet und ist bevorzugt aus einem Kunststoff ausgebildet. Um die Öffnungen 22 herum kann auf den Boden 21 ein elastisches Material, beispielsweise ein Gummi oder weichelastischer Kunststoff, ringförmig aufgebracht werden, um als Dichtung zu wirken, wie nachfolgend anhand der Figuren 8c-8e beschrieben. Hierzu eignen sich insbesondere 2K-Spritzgussverfahren. Ein solches Dichtungselement kann auch als gesonderter Dichtungsring um die Öffnungen 22 herum auf den Boden 21 des Trägers 20 aufgelegt werden.

Gemäß der Fig. 8a ist der Träger 20 trogförmig ausgebildet, mit einem vergleichsweise breiten Rand 40, der über leicht einwärts geneigt Seitenwände 35 mit dem Boden 21 verbunden ist. Auf den Innenseiten der Seitenwände 35 sind Rippen 46 ausgebildet, die als Abstandshalter dienen, wenn mehrere solche Träger 20 übereinander gestapelt aufbewahrt werden.

Die Fig. 8b zeigt den in der Fig. 8a eingekreisten Ausschnitt in einer stark vergrößerten Darstellung.

Anhand der Figuren 8c bis 8e wird nachfolgend ein Verfahren gemäß der vorliegenden Erfindung beschrieben, mit dem das hintere Ende einer Karpule (Zylinderampulle) mit einem Gummistopfen verschlossen wird. Es sei ausdrücklich daraufhingewiesen, dass dieses Verfahren auch außerhalb eines Trägers, wie vorstehend beschrieben, ausgeführt werden kann und als Verfahren eine eigenständige Erfindung darstellt, die gemäß der vorliegenden Anmeldung ausdrücklich auch unabhängig zu den vorstehenden Ausführungsformen beansprucht werden kann.

Das Befüllen und Verschließen vorsterilisierter Karpulen ist von besonderem Interesse, da sichergestellt werden muss, dass das in der Karpule aufbewahrte Medikament seine Eigenschaften über Jahre beibehält. Um eine Wechselwirkung des Medikaments mit Luft zu verhindern, wird die Karpule erfindungsgemäß evakuiert und anschließend ein Stopfen in das hintere Ende der Karpule eingeführt, um dieses zu Verschließen.

Gemäß der Fig. 8c wird der Stopfen 80 von einem Vakuum-Greifer 70 in einem komprimierten Zustand in einen Zylinder 71 eingeführt und in dem Zylinder 71 komprimiert gehalten. Der Außendurchmesser des Zylinders 71 ist kleiner als der Innendurchmesser der Karpule 2. Ferner ist der Innendurchmesser des Zylinders 71 kleiner als der Außendurchmesser des Stopfens 80, wenn dieser nicht komprimiert ist. Der Vakuum-Greifer 70 kann gemeinsam mit dem Zylinder 71 axial verstellt werden, jedoch auch relativ zu dem Zylinder 71 axial verstellt werden.

In die Karpule 2, von der in den Figuren 8c bis 8e nur das hintere Ende dargestellt ist, ist eine Flüssigkeit 9a eingefüllt. Oberhalb des Flüssigkeitsspiegels 9c der Flüssigkeit 9a ist das Innenvolumen 9b der Karpule 2 nicht gefüllt. Gemäß der Fig. 8c ist auf das hintere Ende der Karpule 2, das bei diesem Ausführungsbeispiel flach ausgebildet ist (also Fingergriff), ein ringförmiges Dichtungselement 77 aufgesetzt, um das hintere Ende der Karpule 2 gegen die Umgebung abzudichten. In die Öffnung des Dichtungselements 77 kann der Zylinder 71 mit dem Vakuum-Greifer 70 eingeführt werden, weil der Außendurchmesser des Zylinders 71 kleiner als der Durchmesser der Öffnung ist.

Am vorderen Ende des Zylinders 71 sitzt ein Druckstempel 73, der über einen Dichtungsring 74 gegen den Zylinder 71 abgedichtet ist. Zylinder 71 und Vakuum-Greifer 70 können axial relativ zu dem Druckstempel 73 verstellt werden. Der Druckstempel 73 dient als Anlagefläche, die an dem Dichtungselement 77 anliegt und dieses mit einem Druck beaufschlagen kann, um das Innenvolumen der Karpule gegen die Umgebung abzudichten. Gemäß der Fig. 8c ist in dem Druckstempel 77 ein radialer Absaugkanal 75 ausgebildet, über den mittels einer Absaugeinrichtung (nicht gezeigt) Luft oder Gas aus dem Ringspalt 76 zwischen dem Zylinder 71 und der Karpule 2 abgesaugt werden kann. Das Dichtungselement 77 kann aus einem elastischen Gummimaterial bestehen und auf den hinteren Rand der Karpule 2 als separates Element aufgesetzt werden. Bei dem Dichtungselement kann es sich jedoch auch um den Boden 21 eines Trägers 20 (vgl. Fig. 8a) oder um das um die jeweilige Öffnung 22 herum im Boden 21 des Trägers 20 angeordnete oder aufgebrachte Dichtungselement handeln. Wenn die Karpulen aufrecht auf dem Boden 21 eines Trägers 20 aufliegen, der wie in der Fig. 8a gezeigt geschlossen ausgebildet ist, kann die in der Fig. 8c gezeigte Dichtungsanordnung automatisch realisiert werden, wenn die Karpulen 2 in einen Träger aufrecht eingesetzt sind. Bei dieser Stellung kann es sich insbesondere auch um die Transportstellung handeln, in der die Karpulen 2 in einen Träger (Nest) eingesetzt sind und in der der Träger in einem Transport- und Verpackungsbehälter steril aufbewahrt und transportiert wird, wie dieser beispielsweise in der Fig. 7d dargestellt ist.

Der Verschließ-Vorgang beginnt mit dem Setzen der Stopfen: Ein Sortiertopf (nicht gezeigt) führt dem Vakuum-Greifer 70 Stopfen 80 zu, der diese im Anschluss der Karpule aufdrücken soll. Der Vakuum-Greifer 70 drückt den Stopfen 80 in den Zylinder 71 hinein, wobei der Stopfen 80 dabei komprimiert wird, wie in der Fig. 8c gezeigt. Anschließend wird der Vakuum-Greifer 70 mit dem Zylinder 71 und dem Druckstempel 73 auf den hinteren Rand der Karpule 2 aufgesetzt, wobei dabei das Dichtungselement 77 zwischen dem Druckstempel 73 und dem hinteren Rand der Karpule 2 eingeklemmt wird, um das nicht mit Flüssigkeit gefüllte Innenvolumen 9b und den Ringspalt 76 zwischen dem Zylinder 71 und der Karpule 2 gegen die Umgebung abzudichten.

Über den Absaugkanal 75 wird anschließend die Luft aus dem nicht mit Flüssigkeit gefüllten Innenvolumen 9b abgesaugt. Anschließend wird der Vakuum-Greifer 70 relativ zu dem Zylinder 71 axial hin zu dem Flüssigkeitsspiegel 9c verstellt. Dabei wird der Stopfen 80 zunächst aus dem Zylinder 71 gedrückt, sodass dieser sich entspannt und dichtend an der Innenwand der Karpule 2 anliegt. Anschließend wird der Vakuum-Greifer 70 relativ zu dem Zylinder 71 weiter axial hin zu dem Flüssigkeitsspiegel 9c verstellt, bis der Stopfen 80 auf dem Flüssigkeitsspiegel 9c aufliegt, wie in der Fig. 8d gezeigt. Nach Trennen des Vakuum-Greifers 70 von dem Stopfen 80 kann die Dichtungsanordnung wieder aufgelöst werden und können der Vakuum-Greifer 70 und der Zylinder 71 wieder axial aus dem hinteren Ende der Karpule 2 herausgefahren werden, wie in der Fig. 8d gezeigt.

Anschließend wird der Vakuum-Greifer 70 weiter relativ zu dem Zylinder 71 axial verstellt, wie in der Fig. 8e gezeigt, und wird der Vakuum-Greifer 70 gemeinsam mit dem Zylinder 71 axial aus dem hinteren Ende der Karpule 2 herausgefahren, sodass ein neuer Verschließ-Vorgang beginnen kann.

Bei diesem Verschließ-Vorgang kann eine optische Kontrolle überprüfen, ob die Stopfen auch wirklich anwesend sind. Ferner können die Positionen der Karpule, des Stopfens und/oder des Vakuum-Greifers bestimmt und geeignet nachgeführt werden. In einem weiteren Teil des Verschließ-Vorgangs kann eine Abschleppbahn Verschlusskappen auf den mit Stopfen versehenen Karpulen aufsetzen. Bei den Verschlusskappen kann es sich insbesondere um Metalldeckel handeln, die auf den Rand der Karpulen gebördelt werden. Diese Verschlusskappen kann auch hier ein Sortiertopf zuführen.

Ein Befüllen von Behältern, insbesondere von Karpulen, unter Vakuum, mit dem sich die Blasenbildung verhindern lässt, wird zumeist mit Rotationskolbenpumpen durchgeführt. Die Kunst bei der Verarbeitung viskoser Flüssigkeiten unter Vakuum liegt darin, ein Gleichgewicht der verschiedenen Parameter zu finden. Ein zu tiefes Vakuum kann bei herkömmlichen Verfahren dazu führen, dass das Produkt zu sieden beginnt. Beim herkömmlichen Setzen der Stopfen besteht wiederum die Gefahr, dass Produkt am Stopfen vorbei aus der Karpule abgesaugt wird. Ist das Vakuum jedoch zu gering, lösen sich Lufteinschlüsse oder -blasen nicht auf.

Über die Breite des Ringspalts 76 (vgl. Fig. 8c) und des Absaugkanals 75 sowie den zum Absaugen verwendeten Unterdruck können erfindungsgemäß in einfacher Weise geeignete Parameter festgelegt werden, sodass Stopfen ohne Sieden der Flüssigkeit aber auch ohne Lufteinschlüsse oder Luftblasen in der Flüssigkeit gesetzt werden können. Positiv wirkt sich aus, dass mit Hilfe des vorstehend beschriebenen Verschließ-Vorgangs bei nur wenig Luft in der Karpule ein höheres Sterilitätssicherheitslevel erreicht werden kann, die Dosiergenauigkeit in der Regel höher ist und auch eine mögliche Bewegung des Stopfens bei veränderten klimatischen Bedingungen (Luftdruck) während des Transports ausgeschlossen werden kann.

Der vorgenannte Verschließ-Vorgang kann insbesondere auch so ausgeführt werden, dass die Behälter, insbesondere Karpulen, in einem Träger, wie vorstehend beschrieben, in einer vorbestimmten geometrischen Anordnung einer Verschschließ-Station zugeführt werden, wobei ein offenes Ende der Behälter möglichst nahe zum Boden des Trägers angeordnet ist. Beispielswiese kann das offene Ende eines Behälters unmittelbar auf dem Boden des Trägers abgestützt sein oder das offene Ende, beispielsweise der Hals eines Behälters, kann die Öffnungen im Boden des Trägers bis zur anderen Seite des Trägers durchragen. Damit kann der Boden des Trägers unmittelbar als Gegenelement für den Druckstempel der Verschschließ-Station wirken. Die Behälter sind so an dem Träger gehalten, dass deren offene Enden fluchtend zu den Öffnungen im Boden des Trägers angeordnet sind. Anschließend nähert sich der Druckstempel der Verschschließ-Station dem offenen Ende des Behälters, wie vorstehend beschrieben, und wird der Verschließ-Vorgang ausgeführt, wie vorstehend beschrieben, während die Behälter an dem Träger gehalten sind.

Wie dem Fachmann beim Studium der vorstehenden Beschreibung ohne Weiteres ersichtlich sein wird, können die Behälter auch mittels einer beliebigen anderen form- oder kraftschlüssigen Verbindung an den Haltestrukturen gehalten sein.

Die von den Haltemitteln jeweils auf die Behälter ausgeübte Haltekraft ist ausreichend, um die Behälter zuverlässig an der Haltestruktur zu halten. Insbesondere ist die ausgeübte Haltekraft größer als die Gewichtskraft der Behälter, ggf. mit Inhalt und Verschlussstopfen. Dadurch wird eine zuverlässige Halterung der Behälter an der Haltestruktur gewährleistet. Gleichzeitig können die Behälter in den Öffnungen oder Aufnahmen der Haltestruktur ohne größeren Kraftaufwand verstellt werden, insbesondere axial vorgeschoben oder gedreht werden.

Selbstverständlich kann die Haltestruktur bzw. der Träger) im Sinne der vorliegenden Erfindung auch aus einem thermoplastischen, duroplastischen oder elastomeren Kunststoff ausgebildet sein, wobei zumindest Abschnitte der Haltestruktur bzw. des Trägers mit einer reibreduzierenden Beschichtung versehen sind, um das Einführen und die Entnahme der Behälter zu erleichtern.

## Patentansprüche

1. Haltestruktur zum gleichzeitigen Halten einer Mehrzahl von Behältern (2) einer vorbestimmten Länge, die zur Aufbewahrung von Substanzen für medizinische, pharmazeutische oder kosmetische Anwendungen dienen oder diese enthalten und die obere Enden (6) und untere Enden (8) aufweisen, mit einem Träger (20), der eine Mehrzahl von Haltemitteln (23) aufweist und ausgelegt ist, um die Behälter wahlweise in einer ersten Orientierung oder in einer zweiten Orientierung, die entgegengesetzt zur ersten Orientierung ist, zu halten, **dadurch gekennzeichnet, dass** die Haltestruktur zweiteilig ausgebildet ist und den Träger (20) und einen Rahmen (50) umfasst, an welchem der Träger (20) in einer ersten vorbestimmten Orientierung oder in einer zweiten vorbestimmten Orientierung, die entgegengesetzt zu der ersten vorbestimmten Orientierung ist, abstützbar ist, wobei der Träger (20) so auf die Länge der Behälter (2) abgestimmt ist, dass
die oberen Enden (6) der Behälter (2) in einer ersten Stellung (Fig. 3i), in welcher die Behälter (2) in der ersten Orientierung an dem Träger (20) gehalten sind und der Träger (20) in der ersten vorbestimmten Orientierung an dem Rahmen (50) abgestützt ist, unter dem gleichen Abstand zum oberen Rand des zur Abstützung des Trägers (20) dienenden Rahmens (50) angeordnet sind wie die unteren Enden (3; 8) der Behälter (2) in einer zweiten Stellung (Fig. 3h), in welcher die Behälter (2) in der zweiten Orientierung an dem Träger (20) gehalten sind und der Träger (20) in der zweiten vorbestimmten Orientierung an dem Rahmen (50) abgestützt ist, und
die oberen Enden (6) und/oder unteren Enden (8) der Behälter (2), während diese an dem Träger (20) gehalten sind, für eine Weiterverarbeitung der Behälter zugänglich sind.

2. Haltestruktur zum gleichzeitigen Halten einer Mehrzahl von Behältern (2) einer vorbestimmten Länge, die zur Aufbewahrung von Substanzen für medizinische, pharmazeutische oder kosmetische Anwendungen dienen oder diese enthalten und die obere Enden (6) und untere Enden (8) aufweisen, mit einem Träger (20), der eine Mehrzahl von Haltemitteln (23) aufweist und ausgelegt ist, um die Behälter wahlweise in einer ersten oder zweiten Orientierung an dem Träger (20) zu halten, **dadurch gekennzeichnet, dass** die Haltestruktur zweiteilig ausgebildet ist und den Träger (20) und einen Rahmen (50) aufweist, an welchem der Träger (20) in einer ersten Orientierung oder in einer zweiten Orientierung, die entgegengesetzt zu der ersten Orientierung ist, abstützbar ist, wobei der Träger (20) so auf die Länge der Behälter (2) abgestimmt ist, dass
die oberen Enden (6) der Behälter (2) in einer ersten Stellung (Fig. 4h), in welcher die Behälter (2) in einer vorbestimmten Orientierung an dem Träger (20) gehalten sind und der Träger (20) in der ersten Orientierung an dem Rahmen (50) abgestützt ist, unter dem gleichen Abstand zum oberen Rand des zur Abstützung des Trägers (20) dienenden Rahmens (50) angeordnet sind wie die oberen Enden (6) der Behälter (2) in einer zweiten Stellung (Fig. 4f), in welcher die Behälter (2) in der gleichen vorbestimmten Orientierung an dem Träger (20) gehalten sind und der Träger (20) in der zweiten Orientierung an dem Rahmen (50) abgestützt ist, wobei
die oberen Enden (6) der Behälter (2), während der Träger (20) in der ersten Orientierung an dem Rahmen (50) abgestützt ist und die Behälter (2) in der vorbestimmten Orientierung an dem Träger (20) gehalten sind, für eine Weiterverarbeitung der Behälter zugänglich sind, oder
die oberen Enden (6) der Behälter (2), während der Träger (20) in der zweiten Orientierung an dem Rahmen (50) abgestützt ist und die Behälter (2) in der vorbestimmten Orientierung an dem Träger (20) gehalten sind, für eine Weiterverarbeitung der Behälter zugänglich sind.

3. Haltestruktur nach Anspruch 1 oder 2, wobei der Träger eine Mehrzahl von Öffnungen (22) aufweist, denen jeweils bevorzugt zwei Haltemittel (23) zugeordnet sind, wobei die Behälter (2) so von den Haltemitteln (23) an dem Träger (20) gehalten sind, dass diese sich durch die Öffnungen des Trägers hindurch erstrecken, dass die oberen Enden (6) der Behälter über einen oberen Rand des Trägers vorstehen und/oder dass die unteren Enden (3; 8) der Behälter über einen unteren Rand des Trägers vorstehen.

4. Haltestruktur nach einem der vorhergehenden Ansprüche, wobei die Behälter in der ersten Stellung und in der zweiten Stellung an dem Träger abgestützt sind oder an dem Träger axial gesichert gehalten sind.

5. Haltestruktur nach einem der vorhergehenden Ansprüche, wobei die Haltemittel als elastische Haltezungen (23) ausgebildet sind, die am Rand einer jeweiligen Öffnung vorgesehen sind und von einer Oberseite des Trägers (20) abragen, um den jeweiligen Behälter zu halten, wobei die elastischen Haltezungen (23) so ausgelegt sind, dass diese beim Einführen der Behälter in die Öffnungen oder Aufnahmen elastisch weggeschwenkt oder weggeklappt werden, wobei die elastischen Haltezungen bevorzugt so auf die Behälter abgestimmt sind, dass diese mit radialem Spiel von den elastischen Haltezungen gehalten sind.

6. Haltestruktur nach einem der vorhergehenden Ansprüche, wobei von einer Unterseite des Trägers (20) Seitenwände (37) oder Zapfen (42) im Wesentlichen senkrecht abragen, um eine Berührung von unmittelbar benachbart an dem Träger gehaltenen Behältern zu verhindern, wobei
die Seitenwände (37) oder Zapfen (42) so ausgebildet sind, dass die Behälter von der Unterseite des Trägers (20) her frei zugänglich sind, oder
wobei die Seitenwände (37) nicht umlaufend ausgebildet sind, so dass ein Abschnitt der an dem Träger gehaltenen Behälter seitlich zugänglich ist.

7. Haltestruktur nach einem der Ansprüche 1 bis 4, wobei von einer Unterseite des Trägers (20) Zapfen (42) im Wesentlichen senkrecht abragen, um eine Berührung von unmittelbar benachbart an dem Träger gehaltenen Behältern zu verhindern, wobei die Haltemittel (43) so ausgelegt sind, dass die jeweiligen Enden der Behälter auf den Haltemitteln abgestützt sind, wobei die Haltemittel (43) als sich kreuzende und miteinander verbundene Haltestege ausgebildet sind, wobei zumindest zwei Haltestege (43) jeweils eine in dem Träger ausgebildete Öffnung (22) überbrücken, und wobei im Bereich eines Kreuzungspunkts (44) der sich kreuzenden Haltestege (43) ein vertiefter Abschnitt ausgebildet ist, um eine Aufnahme für einen verengten Halsabschnitt (5, 6) der zu haltenden Behälter auszubilden, um einem Verkippen der an dem Träger gehaltenen Behälter entgegen zu wirken.

8. Haltestruktur nach einem der vorhergehenden Ansprüche, wobei Elemente (54; 40, 41) vorgesehen sind, insbesondere an dem Rahmen (50) und dem Träger (20), um in der jeweiligen Orientierung eine unterschiedliche Höhendifferenz zwischen dem Rahmen (50) und dem Träger (20) festzulegen, wobei die Elemente (54; 40, 41) bevorzugt jeweils formschlüssig miteinander zusammen wirken.

9. Transport- und Verpackungsbehälter für eine Mehrzahl von Behältern (2) für Substanzen für medizinische, pharmazeutische oder kosmetische Anwendungen, wobei der Transport- und Verpackungsbehälter (10) kastenförmig ausgebildet ist, **gekennzeichnet durch** eine Haltestruktur nach einem der vorhergehenden Ansprüche, die in dem kastenförmigen Transport- und Verpackungsbehälter aufgenommen ist, um die Mehrzahl von Behältern (2) in dem Transport- und Verpackungsbehälter zu halten, wobei die Behälter (2) jeweils nicht über einen oberen bzw. unteren Rand des Transport- und Verpackungsbehälters vorstehen, wenn die Behälter an dem Träger in der ersten Stellung oder in der zweiten Stellung gehalten sind.

10. Verfahren zur Behandlung oder Verarbeitung von Behältern (2), die zur Aufbewahrung von Substanzen für medizinische, pharmazeutische oder kosmetische Anwendungen dienen oder diese enthalten und die obere Enden (6) und untere Enden (8) aufweisen, wobei die Behälter an zumindest einem Ende offen ausgebildet sind und wobei die Behälter mittels einer Fördereinrichtung (113) zur Behandlung oder Verarbeitung an Prozessstationen (111, 112) vorbeigeführt werden oder diese durchlaufen, bei dem
eine Mehrzahl von Behältern (2) von der Fördereinrichtung gefördert wird, während diese an einer Haltestruktur gehalten werden, und
die Behälter, während diese an der Haltestruktur gehalten werden, behandelt oder verarbeitet werden, **dadurch gekennzeichnet dass**
die Haltestruktur zweiteilig ausgebildet ist und einen Träger (20) und einen Rahmen (50) umfasst, an welchem der Träger (20) wahlweise in einer ersten vorbestimmten Orientierung oder in einer zweiten vorbestimmten Orientierung, die entgegengesetzt zu der ersten vorbestimmten Orientierung ist, abgestützt werden kann, und
die Behälter wahlweise in einer ersten Orientierung und in einer zweiten Orientierung, die entgegengesetzt zur ersten Orientierung ist, an dem Träger (20) gehalten werden können,
bei welchem Verfahren
die oberen Enden (6) der Behälter (2) in einer ersten Stellung (Fig. 3i), in welcher die Behälter (2) in der ersten Orientierung an dem Träger (20) gehalten werden und der Träger (20) in der ersten vorbestimmten Orientierung an dem Rahmen (50) abgestützt ist, unter dem gleichen Abstand zum oberen Rand des Trägers (20) oder zum oberen Rand (53) des zur Abstützung des Trägers (20) dienenden Rahmens (50) angeordnet sind wie die unteren Enden (3; 8) der Behälter (2) in einer zweiten Stellung (Fig. 3h), in welcher die Behälter (2) in der zweiten Orientierung an dem Träger (20) gehalten werden und der Träger (20) in der zweiten vorbestimmten Orientierung an dem Rahmen (50) abgestützt ist, und
zur Behandlung oder Verarbeitung auf die Behälter (2) über deren obere Enden (6) und/oder über deren untere Enden (8) in der ersten oder zweiten Stellung eingewirkt wird.

11. Verfahren zur Behandlung oder Verarbeitung von Behältern (2), die zur Aufbewahrung von Substanzen für medizinische, pharmazeutische oder kosmetische Anwendungen dienen oder diese enthalten und die obere Enden (6) und untere Enden (8) aufweisen, wobei die Behälter an zumindest einem Ende offen ausgebildet sind und wobei die Behälter mittels einer Fördereinrichtung (113) zur Behandlung oder Verarbeitung an Prozessstationen (111, 112) vorbeigeführt werden oder diese durchlaufen, bei dem
eine Mehrzahl von Behältern (2) von der Fördereinrichtung gefördert wird, während diese an einer Haltestruktur gehalten werden, und
die Behälter, während diese an der Haltestruktur gehalten werden, behandelt oder verarbeitet werden, **dadurch gekennzeichnet dass**
die Haltestruktur zweiteilig ausgebildet ist und einen Träger (20) und einen Rahmen (50) umfasst, an welchem der Träger (20) wahlweise in einer ersten Orientierung oder in einer zweiten Orientierung, die entgegengesetzt zu der ersten Orientierung ist, abgestützt werden kann, und
der Träger (20) wahlweise in einer ersten Orientierung oder in einer zweiten Orientierung an dem Rahmen (50) gehalten werden kann,
bei welchem Verfahren
die oberen Enden (6) der Behälter (2) in einer ersten Stellung (Fig. 4h), in welcher die Behälter (2) in einer vorbestimmten Orientierung an dem Träger (20) gehalten werden und der Träger (20) in der ersten Orientierung an dem Rahmen (50) abgestützt ist, unter dem gleichen Abstand zum oberen Rand des zur Abstützung des Trägers (20) dienenden Rahmens (50) angeordnet sind wie die oberen Enden (6) der Behälter (2) in einer zweiten Stellung (Fig. 4f), in welcher die Behälter (2) in der gleichen vorbestimmten Orientierung an dem Träger (20) gehalten werden und der Träger (20) in der zweiten Orientierung an dem Rahmen (50) abgestützt ist, und
zur Behandlung oder Verarbeitung auf die Behälter (2) über deren obere Enden (6) eingewirkt wird, während der Träger (20) in der ersten Orientierung an dem Rahmen (50) abgestützt ist und die Behälter (2) in der vorbestimmten Orientierung an dem Träger (20) gehalten werden,
zur Behandlung oder Verarbeitung auf die Behälter (2) über deren obere Enden (6) und/oder deren untere Enden (8) eingewirkt wird, während der Träger (20) in der zweiten Orientierung an dem Rahmen (50) abgestützt ist und die Behälter (2) in der vorbestimmten Orientierung an dem Träger (20) gehalten werden.

12. Verfahren nach Anspruch 10 oder 11, wobei die Behandlung oder Verarbeitung der Behälter einen Schritt zur Messung einer Füllmenge der Behälter (2) umfasst, bei dem die Behälter (2) so an der Haltestruktur gehalten sind, dass ein Laserstrahl (101) jeweils einen verengten Halsabschnitt (5) der Behälter (2) durchstrahlt und der Laserstrahl (101) mit Hilfe eines Sensors (102) detektiert wird, wobei auf Grundlage eines Messsignals des Sensors (102) auf die jeweilige Füllmenge der Behälter geschlossen wird, insbesondere auf einen Füllstand der Behälter.

13. Verfahren nach einem der Ansprüche 10 bis 12, wobei die Behandlung oder Verarbeitung der Behälter, während diese an dem Träger (20) gehalten werden, mindestens einen Schritt zum Reinigen der Behälter (2) und/oder zum Wiegen der Behälter und/oder zum Befüllen der Behälter (2) und/oder zum Verschließen der Behälter mit Stopfen (80) oder Kappen und/oder zum Bördeln von Metallkappen auf die Behälter umfasst.

14. Verfahren nach Anspruch 13, wobei das Verschließen eines Behälters mit einem Stopfen (80) umfasst:
Anordnen eines Dichtungselements (77; 21) auf einem Ende des Behälters (2), um das Ende des Behälters gegen die Umgebung abzudichten;
Einführen des Stopfens (80) in einem komprimierten Zustand mittels einer Greifeinrichtung (70, 71) in das Ende des Behälters;
Anlegen eines Unterdrucks an das Ende des Behälters über einen Ringspalt (76) zwischen der Greifeinrichtung und einer Innenseite des Behälters; und
Entspannen des Stopfens (80), bis dieser an der Innenseite des Behälters anliegt, um das Ende des Behälters gegen die Umgebung abzudichten.

15. Verfahren nach Anspruch 14, wobei die Greifeinrichtung einen Zylinder (71) umfasst, dessen Außendurchmesser kleiner ist als der Innendurchmesser am Ende des Behälters (2), wobei das Einführen des Stopfens (80) in dem komprimierten Zustand mittels der Greifeinrichtung (70, 71) in das Ende des Behälters weiterhin umfasst:
Komprimieren des Stopfens (80) durch Einführen des Stopfens in den Zylinder (71);
Verstellen des Zylinders (71) gemeinsam mit der Greifeinrichtung (70), um den Stopfen in das Ende des Behälters einzuführen;
Drücken des Dichtungselements (77; 21), um das Ende des Behälters gegen die Umgebung abzudichten; und
Herausschieben des Stopfens (80) aus dem Zylinder (71) weiter in das Ende des Behälters hinein und hin zur Oberfläche (9c) der Flüssigkeit, die in dem Behälter (2) aufbewahrt ist.

16. Verfahren nach Anspruch 14 oder 15, wobei das Dichtungselement ein Boden (21) des Trägers (20) ist, wobei die Behälter (2) so auf dem Boden (21) des Trägers (20) angeordnet werden, dass die Enden der Behälter mit Öffnungen (22), die in dem Boden (21) ausgebildet sind, fluchten und die Stopfen (80) durch die Öffnungen (22) in die Enden der Behälter eingeführt werden.

## Claims

1. A supporting structure for concurrently holding a plurality of containers (2) having a predetermined length, which are used for the storage of substances for medical, pharmaceutical or cosmetic applications or contain such substances and which have upper ends (6) and lower ends (8), comprising a carrier (20) having a plurality of supporting means (23) and being configured to support the containers optionally in a first orientation or in a second orientation opposite to the first orientation, **characterized in that** the supporting structure is formed in two parts and comprises the carrier (20) and a frame (50) on which the carrier (20) can be supported in a first predetermined orientation or in a second predetermined orientation opposite to the first predetermined orientation, wherein the carrier (20) is matched in such a manner to the lengths of the containers (2)
that the upper ends (6) of the containers (2) are arranged in a first position (Fig. 3i), in which the containers (2) are supported on the carrier (20) in the first orientation and the carrier (20) is supported on the frame (50) in the first predetermined orientation, at the same distance to the upper rim of the frame (50) used for supporting the carrier (20) as the lower ends (3; 8) of the containers (2) in a second position (Fig. 3h), in which the containers (2) are supported on the carrier (20) in the second orientation and the carrier (20) is supported on the frame (50) in the second predetermined orientation, and
that the upper ends (6) and/or lower ends (8) of the containers (2) are accessible for a further processing of the containers, while they are supported on the carrier (20).

2. A supporting structure for concurrently holding a plurality of containers (2) having a predetermined length, which are used for the storage of substances for medical, pharmaceutical or cosmetic applications or contain such substances and which have upper ends (6) and lower ends (8), comprising a carrier (20) having a plurality of supporting means (23) and being configured to support the containers optionally in a first orientation or in a second orientation, **characterized in that** the supporting structure is formed in two parts and comprises the carrier (20) and a frame (50) on which the carrier (20) can be supported in a first orientation or in a second orientation opposite to the first orientation, wherein the carrier (20) is matched in such a manner to the lengths of the containers (2)
that the upper ends (6) of the containers (2) are arranged in a first position (Fig. 4h), in which the containers (2) are supported on the carrier (20) in a predetermined orientation and the carrier (20) is supported on the frame (50) in the first orientation, at the same distance to the upper rim of the frame (50) used for supporting the carrier (20) as the upper ends (6) of the containers (2) in a second position (Fig. 4f), in which the containers (2) are supported on the carrier (20) in the same predetermined orientation and the carrier (20) is supported on the frame (50) in the second orientation, wherein
the upper ends (6) of the containers (2) are accessible for a further processing of the containers, while the carrier (20) is supported on the frame (50) in the first orientation and the containers (2) are supported on the carrier (20) in the predetermined orientation, or
the upper ends (6) of the containers (2) are accessible for a further processing of the containers, while the carrier (20) is supported on the frame (50) in the second orientation and the containers (2) are supported on the carrier (20) in the predetermined orientation.

3. The supporting structure according to claim 1 or 2, wherein the carrier comprises a plurality of apertures (22), wherein preferably respective two supporting means (23) are associated to the apertures, wherein the containers (2) are supported by the supporting means (23) on the carrier (20) such that they extend through the apertures of the carrier, that the upper ends (6) of the containers protrude beyond an upper rim of the carrier and/or that the lower ends (3; 8) of the containers protrude beyond a lower rim of the carrier.

4. The supporting structure according to any of the preceding claims, wherein in the first position and in second position the containers are supported on the carrier or retained in axial direction on the carrier.

5. The supporting structure according to any of the preceding claims, wherein the supporting means are designed as supporting tongues (23) that are provided on the edge of a respective aperture and protrude from an upper surface of the carrier (20) to support the respective container, wherein the supporting tongues (23) are configured such that they are elastically pivoted away or flapped away during insertion of the containers into the apertures or receptacles, and wherein the supporting tongues are matched to the containers such that the containers are supported by the supporting tongues with a radial clearance.

6. The supporting structure according to any of the preceding claims, wherein side walls (37) or pins (42) project from an underside of the carrier (20) substantially perpendicularly to prevent a contact of containers that are supported on the carrier directly adjacent to each other, wherein
the side walls (37) or pins (42) are formed such that the containers are freely accessible from the underside of the carrier (20), or
wherein the side walls (37) are not formed as circumferential side walls, so that a portion of the containers supported on the carrier is accessible from the side of the carrier.

7. The supporting structure according to any of claims 1 to 4, wherein pins (42) project essentially perpendicularly from an underside of the carrier (20) to prevent a collision between containers supported on the carrier directly adjacent to each other, wherein the supporting means (43) are configured such that the respective ends of the containers are supported on the supporting means, wherein the supporting means (43) are formed as intersecting and interconnected supporting webs, wherein at least two supporting webs (43) each span an aperture (22) formed in the carrier, and wherein in the region of a point of intersection (44) of the intersecting supporting webs (43) a recessed portion is formed to form a receptacle for a constricted neck portion (5, 6) of the container to be supported, for preventing a tilting of the containers supported on the carrier.

8. The supporting structure according to any of the preceding claims, wherein members (54; 40, 41) are disposed, particularly on the frame (50) and on the carrier (20), for defining a different height difference between the frame (50) and the carrier (20) in the respective orientation, wherein the members (54; 40, 41) cooperate with each other preferably in a positive-fit manner.

9. A transport and packaging container for a plurality of containers (2), which are used for the storage of substances for medical, pharmaceutical or cosmetic applications or contain such substances, wherein the transport and packaging container (10) is box-shaped, **characterized by** a supporting structure according to any of the preceding claims, which is accommodated in the box-shaped transport and packaging container (10) for supporting the plurality of containers (2) in the transport and packaging container, wherein the containers (2) respectively do not protrude beyond on an upper rim and beyond a lower rim, respectively, of the transport and packaging container when the containers are supported on the carrier in the first position or in the second position.

10. A process for the treatment or processing of containers (2), which are used for the storage of substances for medical, pharmaceutical or cosmetic applications or contain such substances and which have upper ends (6) and lower ends (8), wherein the containers are open at least at one end and wherein the containers are conveyed by a conveying device (113) automated past processing stations (111, 112) or are passed through them for the treatment or processing, wherein
a plurality of containers (2) is conveyed by the conveying device, while they are supported by a carrier, and
the containers are treated or processed while they are supported by the carrier, **characterized in that**
the supporting structure is formed in two parts and comprises a carrier (20) and a frame (50) on which the carrier (20) can be supported in a first predetermined orientation or in a second predetermined orientation opposite to the first predetermined orientation, and
the containers can be supported on the carrier (20) optionally in a first orientation and in a second orientation opposite to the first orientation, in which method
the upper ends (6) of the containers (2) are arranged in a first position (Fig. 3i), in which the containers (2) are supported on the carrier (20) in the first orientation and the carrier (20) is supported on the frame (50) in the first predetermined orientation, at the same distance to the upper rim of the frame (50) used for supporting the carrier (20) as the lower ends (3; 8) of the containers (2) in a second position (Fig. 3h), in which the containers (2) are supported on the carrier (20) in the second orientation and the carrier (20) is supported on the frame (50) in the second predetermined orientation, and
it is acted on the containers (2) for the treatment or processing via their upper ends (6) and/or via their lower ends (8) in the first or second position.

11. A process for the treatment or processing of containers (2), which are used for the storage of substances for medical, pharmaceutical or cosmetic applications or contain such substances and which have upper ends (6) and lower ends (8), wherein the containers are open at least at one end and wherein the containers are conveyed by a conveying device (113) automated past processing stations (111, 112) or are passed through them for the treatment or processing, wherein
a plurality of containers (2) is conveyed by the conveying device, while they are supported by a carrier, and
the containers are treated or processed while they are supported by the carrier, **characterized in that**
the supporting structure is formed in two parts and comprises a carrier (20) and a frame (50) on which the carrier (20) can be supported optionally in a first orientation or in a second orientation opposite to the first orientation, and
the carrier (20) can be supported on the frame (50) optionally in a first orientation or in a second orientation,
in which method
the upper ends (6) of the containers (2) are arranged in a first position (Fig. 4h), in which the containers (2) are supported on the carrier (20) in a predetermined orientation and the carrier (20) is supported on the frame (50) in the first orientation, at the same distance to the upper rim of the frame (50) used for supporting the carrier (20) as the upper ends (6) of the containers (2) in a second position (Fig. 4f), in which the containers (2) are supported on the carrier (20) in the same predetermined orientation and the carrier (20) is supported on the frame (50) in the second orientation, and
it is acted on the containers (2) for the treatment or processing via their upper ends (6), while the carrier (20) is supported on the frame (50) in the first orientation and the containers (2) are supported on the carrier (20) in the predetermined orientation,
it is acted on the containers (2) for the treatment or processing via their upper ends (6) and/or via their lower ends (8), while the carrier (20) is supported on the frame (50) in the second orientation and the containers (2) are supported on the carrier (20) in the predetermined orientation.

12. The process according to claim 10 or 11, wherein the treatment or processing of the containers comprises a step for measuring filling capacities of the containers (2), where the containers (2) are supported on the supporting structure in such a manner that a laser beam (101) respectively propagates through a constricted neck portion (5) of the containers (2) and the laser beam (101) is detected using a sensor (102), wherein the respective filling capacities of the containers, in particular a filling level of the containers, is determined on the basis of a measurement signal of the sensor (102).

13. The process according to any of claims 10 to 12, wherein the treatment or processing of the containers, while the containers are supported on the carrier (20), comprises at least one step for cleaning the containers (2) and/or for weighing the containers and/or for filling the containers (2) and/or for sealing the containers with stoppers (80) or caps and/or for crimping metal caps on the containers.

14. The process according to claim 13, wherein the sealing the containers with a stopper (80) comprises:
disposing a sealing member (77; 21) on an end of the containers (2) for sealing the end of the container against the environment;
inserting the stopper (80) in a compressed condition by means of a gripping device (70, 71) into the end of the container;
applying a vaccum to the end of the container via an annular gap (76) between the gripping device and an inner surface of the container; and
releasing the stopper (80) until it abuts against the inner surface of the container, for sealing the end of the container against the environment.

15. The process according to claim 14, wherein the gripping device comprises a cylinder (71) having an outer diameter which is smaller than the inner diameter at the end of the container (2), wherein inserting the stopper (80) in the compressed condition by means of the gripping device (70, 71) into the end of the container further comprises:
compressing the stopper (80) by inserting the stopper into the cylinder (71);
displacing the cylinder (71) together with the gripping device (70) for inserting the stopper into the end of the container;
pushing the sealing member (77; 21), for sealing the end of the container against the environment; and
pushing the stopper (80) out of the cylinder (71) and further into the end of the container and toward a surface (9c) of the liquid stored in the container (2).

16. The process according to claim 14 or 15, wherien the sealing member is a bottom (21) of the carrier (20), wherein the containers (2) are disposed in such a manner on the bottom (21) of the carrier (20) the the ends of the containers are aligned with openings (22) formed in the bottom (21) and that the stoppers (80) are inserted into the ends of the containers via the openings (22).

## Revendications

1. Une structure de support pour maintenir simultanément une pluralité de récipients (2) d'une longueur prédéterminée, qui est utilisée pour le stockage de substances pour des applications cosmétiques, pharmaceutiques ou médicales ou contenant de telles substances et qui disposent d'extrémités supérieures (6) et des extrémités inférieures (8), comprenant un support (20) ayant une pluralité de moyens de support (23) et qui est configuré pour maintenir les récipients optionnellement dans une première orientation ou une seconde orientation opposée à la première orientation, **caractérisée en ce que** la structure de support est formée en deux parties et comporte le support (20) et un cadre (50) sur lequel le support (50) peut être maintenu dans une première orientation prédéfinie ou dans une seconde orientation prédéfinie opposée à la première orientation prédéfinie, dans laquelle le support (20) est adapté aux longueurs des récipients (2) de telle manière
que les extrémités supérieures (6) des récipients (2) sont disposées dans une première position (Fig. 3i), dans laquelle les récipients sont maintenus sur le support (20) dans la première orientation et le support (20) est maintenu sur le cadre (50) avec la première orientation prédéterminée, à la même distance du bord supérieur du cadre (50) utilisé pour maintenir le support (20) que celle des extrémités inférieures (3 ; 8) des récipients (2) dans une seconde position (Fig. 3h), dans laquelle les récipients (2) sont maintenus sur le support (20) dans la seconde orientation et le support (20) est maintenu sur le cadre (5à) dans la seconde orientation prédéterminée, et **en ce que**
les extrémités supérieures (6) et/ou les extrémités inférieures (8) des récipients (2) sont accessible pour un traitement ultérieur des récipients, alors qu'ils sont maintenus sur le support (20).

2. Une structure de support pour maintenir simultanément une pluralité de récipients (2) d'une longueur prédéterminée, qui est utilisée pour le stockage de substances pour des applications cosmétiques, pharmaceutiques ou médicales ou contenant de telles substances et qui disposent d'extrémités supérieures (6) et des extrémités inférieures (8), comprenant un support (20) ayant une pluralité de moyens de support (23) et qui est configuré pour maintenir les récipients optionnellement dans une première orientation ou une seconde orientation, **caractérisée en ce que** la structure de support est formée en deux parties et comporte le support (20) et un cadre (50) sur lequel le support (50) peut être maintenu dans une première orientation prédéfinie ou dans une seconde orientation prédéfinie opposée à la première orientation prédéfinie, dans laquelle le support (20) est adapté aux longueurs des récipients (2) de telle manière
que les extrémités supérieures (6) des récipients (2) sont disposées dans une première position (Fig. 4h), dans laquelle les récipients sont maintenus sur le support (20) dans une première orientation prédéterminée et le support (20) est maintenu sur le cadre (50) avec la première orientation, à la même distance du bord supérieur du cadre (50) utilisé pour maintenir le support (20) que celle des extrémités inférieures (6) des récipients (2) dans une seconde position (Fig. 4f), dans laquelle les récipients (2) sont maintenus sur le support (20) dans la même orientation prédéterminée et le support (20) est maintenu sur le cadre (5à) dans la seconde orientation, dans laquelle
les extrémités supérieures (6) des récipients (2) sont accessibles pour un traitement ultérieur des récipients, tandis que le support (20) est maintenu sur le cadre (50) dans la première orientation et les récipients (2) sont maintenus sur le support dans l'orientation prédéterminée, ou
les extrémités supérieures (6) des récipients (2) sont accessibles pour un traitement ultérieur des récipients, tandis que le support (20) est maintenu sur le cadre (50) dans la seconde orientation et les récipients (2) sont maintenus sur le support (20) avec l'orientation prédéterminée.

3. La structure de support selon la revendication 1 ou 2, dans lequel le support présente une pluralité d'ouvertures (22), dans laquelle de préférence deux moyens de maintien (23) sont associées aux ouvertures, dans laquelle les récipients (2) sont maintenus par les moyens de maintien (23) sur le support (20) de telle manière qu'ils passent à travers les ouvertures du support à travers, que les extrémités supérieures (6) des récipients font saillie au-delà d'un bord supérieur du support en saillie et / ou les extrémités inférieures (3; 8) des récipients font saillie au-delà d'un bord inférieur du support.

4. La structure de support selon l'une quelconque des revendications précédentes, dans laquelle les récipients sont maintenus sur le support dans la première et dans la seconde position ou sont maintenus sur le support suivant une direction axiale.

5. La structure de support selon l'une quelconque des revendications précédentes, dans laquelle les moyens de maintien sont conçus sous la forme de languettes élastiques de maintien (23) disposées sur le bord d'une ouverture respective et font saillie relativement à une surface supérieure du support (20) pour maintenir le récipient respectif, dans laquelle les languettes de maintien (23) sont conçues de manière pouvoir être élastiquement écartées par pivotement ou rabattues lors de l'insertion du récipient dans les ouvertures ou les réceptacles, dans laquelle les languettes de maintien sont de préférence adaptées aux récipients de telle manière que les récipients puissent être maintenus par les languettes de maintien avec un jeu radial.

6. La structure de support selon l'une quelconque des revendications précédentes, dans laquelle des parois latérales (37) ou des broches (42) se projettent depuis un côté inférieur du support (20) de manière substantiellement perpendiculaire pour empêcher un contact des récipients maintenus sur le support, adjacents les uns aux autres, dans laquelle
les parois latérales (37) ou les broches (42) sont formées de telle manière que les récipients sont librement accessibles par le fond du support (20), ou
dans laquelle les parois latérales (37) ne sont pas formées sous la forme de parois latérales circonférentielles, de telle manière qu'une partie des récipients maintenus sur le support reste accessible depuis le côté du support.

7. La structure de support selon l'une quelconque des revendications 1 à 4, dans laquelle les broches (42) se projettent de manière essentiellement perpendiculaire depuis le fond du support (20) pour empêcher une collision entre des récipients adjacents maintenus sur le support, dans laquelle les moyens de maintien (43) sont confgurés de telle sorte que les extrémités respectives des récipients sont maintenues sur les moyens de maintien, dans laquelle les moyens de maintien (43) sont formés en bandes de support interconnectés et s'intercroisant, dans laquelle au moins deux bandes de support (43) couvrent chacun une ouverture (22) formée sur le support, et dans laquelle l'on forme une partie évidée dans la région d'un point d'intersection (44) des bandes de support s'intercroisant (43) pour former un logement pour une partie de col rétréci (5,6) du récipient à maintenir, pour empêcher l'inclinaison des récipients maintenus sur le support.

8. La structure de support selon l'une quelconque des revendications précédentes, dans laquelle des éléments (54 ; 40, 41) sont disposés, particulièrement sur le cadre (50) et sur le support (20), pour définir une différence de hauteur différente entre le cadre (50) et le support (20) dans l'orientation respective, dans laquelle les éléments (54 ; 40, 41) coopèrent de manière appropriée les uns avec les autres.

9. Un récipient de transport et de conditionnement pour une pluralité de récipients (2), utilisés pour le stockage de substances pour des applications cosmétiques, pharmaceutiques ou médicales ou destinés à contenir de telles substances, dans lequel le récipient de transport et de conditionnement (10) présente la forme d'une boîte, **caractérisé par** une structure de support selon l'une quelconque des revendications précédentes, qui est logé dans le récipient de transport et de conditionnement en forme de boîte (10) pour maintenir la pluralité de récipients (2) dans le récipient de transport et de conditionnement, dans lequel les récipients (2) ne font pas saillie respectivement au delà d'un bord supérieur et d'un bord inférieur, respectivement, du récipient de transport et de conditionnement lorsque les récipients sont maintenus dans la première ou seconde position sur le support.

10. Un procédé de traitement ou transformation de récipients (2), qui sont utilisés pour le stockage de substances pour des applications cosmétiques, pharmaceutiques ou médicales ou destinés à contenir de telles substances, et qui disposent d'extrémités supérieures (6) et d'extrémités inférieures (8), dans lequel les récipients sont ouverts au moins à une extrémité et dans lequel les récipients sont déplacés au moyen d'un dispositif de convoyage (133) entre des stations de traitement automatisées (111, 112) ou transportés entre elles pour traitement ou transformation, dans lequel
une pluralité de récipients (2) est déplacée au moyen du dispositif de convoyage, tandis qu'ils sont maintenus par un support, et
les récipients sont traités ou transformés tandis qu'ils sont maintenus par le support
**caractérisé en ce que**
la structure de support est formée en deux parties et comporte un support (20) et un cadre (50) sur lequel le support (50) peut être maintenu dans une première orientation prédéfinie ou dans une seconde orientation prédéfinie opposée à la première orientation prédéfinie, et
les récipients peuvent être maintenus sur le support (20) optionnellement dans une première orientation et une seconde orientation opposée à la première orientation, dans lequel procédé :
les extrémités supérieures (6) des récipients (2) sont disposées dans une première position (Fig. 3i), dans laquelle les récipients (2) sont maintenus sur le support (20) dans la première orientation et le support (20) est maintenu sur le cadre (50) avec la première orientation prédéterminée, à la même distance du bord supérieur du cadre (50) utilisé pour maintenir le support (20) que celle des extrémités inférieures (3 ; 8) des récipients (2) dans une seconde position (Fig. 3h), dans laquelle les récipients (2) sont maintenus sur le support (20) dans la seconde orientation et le support (20) est maintenu sur le cadre (50) dans la seconde orientation prédéterminée, et
est mise en oeuvre sur les récipients (2) pour le traitement ou la transformation via leurs extrémités supérieures (6) et/ou via leurs extrémités inférieures (8) dans la première ou seconde position.

11. Un procédé pour la transformation ou le traitement de récipients (2), qui sont utilisés pour le stockage de substances pour des applications cosmétiques, pharmaceutiques ou médicales ou destinés à contenir de telles substances, et qui disposent d'extrémités supérieures (6) et d'extrémités inférieures (8), dans lequel les récipients sont ouverts au moins à une extrémité et dans lequel les récipients sont déplacés au moyen d'un dispositif de convoyage (133) entre des stations de traitement automatisées (111, 112) ou transportés entre elles pour traitement ou transformation, dans lequel
une pluralité de récipients (2) est déplacée par le dispositif de convoyage, tandis qu'ils sont maintenus par un support, et
les récipients sont traités ou transformés tandis qu'ils sont maintenus par le support
**caractérisé en ce que**
la structure de support est formée en deux parties et comporte a support (20) et un cadre (50) sur lequel le support (50) peut être optionnellement maintenu dans une première orientation prédéfinie ou dans une seconde orientation prédéfinie opposée à la première orientation prédéfinie, et
le support (20) peut être maintenu sur le cadre (50) optionnellement dans une première orientation ou dans une seconde orientation,
dans lequel procédé :
les extrémités supérieures (6) des récipients (2) sont disposées dans une première position (Fig. 4h), dans laquelle les récipients (2) sont maintenus sur le support (20) dans une première orientation prédéterminée et le support (20) est maintenu sur le cadre (50) avec la première orientation, à la même distance du bord supérieur du cadre (50) utilisé pour maintenir le support (20) que celle des extrémités inférieures (6) des récipients (2) dans une seconde position (Fig. 4f), dans laquelle les récipients (2) sont maintenus sur le support (20) dans la même orientation prédéterminée et le support (20) est maintenu sur le cadre (50) dans la seconde orientation, et
est mise en oeuvre sur les récipients pour le traitement ou transformation via leurs extrémités supérieures (6), tandis que le support (20) est maintenu sur le cadre (50) dans la première orientation et les récipients (2) sont maintenus sur le support (20) dans l'orientation prédéterminée, ou
est mis en oeuvre sur les récipients pour le traitement ou transformation via leurs extrémités supérieures (6) des récipients (2) et/ou via leurs extrémités inférieures (8), tandis que le support est maintenu sur le cadre (50) dans la seconde orientation et les récipients (2) sont maintenus sur le support (20) avec l'orientation prédéterminée.

12. Le procédé selon la revendication 10 ou 11, dans lequel le traitement ou la transformation des récipients comportent une étape de mesure des capacités de remplissage des récipients (2), dans lequel les récipients (2) sont maintenus sur la structure de support de telle manière qu'un faisceau laser (101) propage respectivement au travers une portion de col rétréci (5) des récipients et le rayon laser (101) est détecté au moyen d'un capteur (102), dans lequel les capacités de remplissage respective des récipients , en particulier un niveau de remplissage des récipients, est déterminé sur la base d'un signal de mesure du capteur (102).

13. Le procédé selon l'une quelconque des revendications 10 à 12, dans lequel le traitement ou la transformation des récipients, pendant qu'ils sont sur le support (20), comportent au moins une étape de nettoyage des récipients (2) et / ou de pesée des récipients et / ou de remplissage des récipients (2) et / ou de fermeture des récipients avec un bouchon (80) ou capuchon et / ou d'évasement des capsules métalliques composant le récipient.

14. Le procédé selon la revendication 13, dans lequel ladite fermeture d'un récipient avec un bouchon (80) comporte :
la mise en place d'un élément d'étanchéité (77; 21) à une extrémité des récipients (2) pour obturer l'extrémité des récipient vis-à-vis de leur environnement;
l'insertion du bouchon (80) en situation de compression au moyen d'un dispositif de préhension (70, 71) dans l'extrémité du récipient;
l'application de vide à l'extrémité du récipient par l'intermédiaire d'une fente annulaire (76) entre le dispositif de préhension et une surface interne du récipient; et
la libération du bouchon (80) jusqu'à ce qu'il vienne en butée contre la surface interne du récipient, pour le scellement de l'extrémité du récipient vis-à-vis de son environnement.

15. Le procédé selon la revendication 14, dans lequel le dispositif de préhension comprend un cylindre (71) dont le diamètre extérieur est inférieur au diamètre interne à l'extrémité du récipient (2), dans lequel l'insertion du bouchon (80) en situation de compression au moyen d'un dispositif de préhension (70, 71) dans l'extrémité du récipient comporte en outre :
la compression du bouchon (80) en insérant le bouchon dans le cylindre (71);
le déplacement du cylindre (71) conjointement avec les moyens de préhension (70) pour l'introduction du bouchon dans l'extrémité du récipient;
le pressage de l'élément d'étanchéité (77; 21) pour sceller l'extrémité du récipient vis à vis de l'environnement; et
le fait de pousser le bouchon (80) hors du cylindre (71) et plus en avant dans l'extrémité du récipient jusqu'à une surface (9c) du liquide qui est stocké dans le récipient (2).

16. Le procédé selon la revendication 14 ou 15, dans lequel l'élément d'étanchéité est une base (21) du support (20), dans lequel les récipients (2) sont disposés sur le fond (21) du support (20) de telle sorte que les extrémités des récipients sont alignées avec des ouvertures (22) qui sont formées dans le fond (21) et les bouchons (80) sont insérés dans les extrémités du récipient à travers les ouvertures (22).
